(19) Europäisches Patentamt
European Patent Office
Office européen des brevets

(11) Veröffentlichungsnummer: **0 455 596 B1**

(12) # EUROPÄISCHE PATENTSCHRIFT

(45) Veröffentlichungstag der Patentschrift: **02.11.94**

(51) Int. Cl.$^5$: **C07D 209/40**, A61K 31/40

(21) Anmeldenummer: **91810313.6**

(22) Anmeldetag: **25.04.91**

Die Akte enthält technische Angaben, die nach dem Eingang der Anmeldung eingereicht wurden und die nicht in dieser Patentschrift enthalten sind.

(54) **Substituierte Indole.**

(30) Priorität: **04.05.90 CH 1510/90**

(43) Veröffentlichungstag der Anmeldung:
**06.11.91 Patentblatt 91/45**

(45) Bekanntmachung des Hinweises auf die Patenterteilung:
**02.11.94 Patentblatt 94/44**

(84) Benannte Vertragsstaaten:
**AT BE CH DE DK ES FR GB GR IT LI LU NL SE**

(56) Entgegenhaltungen:
**EP-A- 0 199 543**
**EP-A- 0 227 241**

**Journal of Medicinal Chemistry, Volume 33, Number 6, June 1990, Seiten 1781-90, Victor G. Matassa et al.**

(73) Patentinhaber: **CIBA-GEIGY AG**
**Klybeckstrasse 141**
**CH-4002 Basel (CH)**

(72) Erfinder: **Sallmann, Alfred Dr.**
**Joachimsackerstrasse 12**
**CH-4103 Bottmingen (CH)**
Erfinder: **Meyer, Willy**
**Talweg 49**
**CH-4125 Riehen (CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 455 596 B1

**Beschreibung**

In EP-A-199 543 werden Indol-Derivate offenbart, die Leukotrien-hemmende Eigenschaften aufweisen. Insbesondere werden darin in den Beispielen 105 und 57 das 2-Methylbenzol- sowie das Benzolsulfonsäure-N-[4-(5-cyclopentyloxycarbonylamino-1-methyl-indol-3-ylmethyl)-3-methoxy-benzoyl]amid beschrieben.

In EP-A-227 241 werden ebenfalls Indol-Derivate offenbart, die Leukotrien-hemmende Eigenschaften aufweisen. Diese unterscheiden sich von denjenigen aus EP-A-199 543 nur dadurch, dass anstelle des Benzolsulfonsaure-amidstickstoffs (-NH-) eine $-CH_2$-Gruppe vorliegt (siehe Beispiel 7 darin).

Die Erfindung betrifft neue trisubstituierte Indol-Verbindungen der Formel

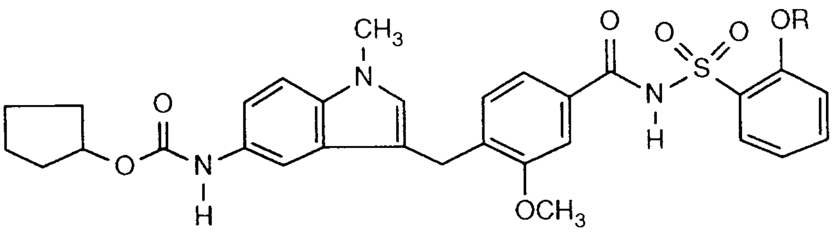 (I),

worin R geradkettiges $C_2$-$C_4$-Alk-1-en-1-yl bedeutet, und ihre Salze, die Verwendung dieser Verbindungen und Salze, ein Verfahren zur Herstellung dieser Verbindungen und Salze und pharmazeutische Zusammensetzungen, enthaltend eine solche Verbindung I in freier Form oder in Form eines pharmazeutisch verwendbaren Salzes.

Die Verbindungen I können im Rahmen der Erfindung, wenn der Rest R mehr als zwei Kohlenstoffatome (C-Atome) besitzt und daher (E)- oder (Z)-Konfiguration aufweisen kann, in Form von Stereoisomeren, z. B. als reine Diastereomere oder Diastereomerengemische, vorliegen. Bevorzugt sind im Rahmen der Erfindung Verbindungen I, in welchen der Rest R die beispielsgemäß offenbarte Stereochemie aufweist.

Salze von Verbindungen I sind insbesondere pharmazeutisch verwendbare Salze, z. B. Säureadditionssalze, die beispielsweise mit starken anorganischen Säuren, wie Mineralsäuren, z. B. Schwefelsäure, einer Phosphorsäure oder einer Halogenwasserstoffsäure, mit starken organischen Carbonsäuren, wie Niederalkancarbonsäuren, z. B. Essigsäure, wie gegebenenfalls ungesättigten Dicarbonsäuren, z.B. Malon-, Malein- oder Fumarsäure, oder wie Hydroxycarbonsäuren, z.B. Wein- oder Zitronensäure, oder mit Sulfonsäuren, wie Niederalkan- oder gegebenenfalls substituierten Benzolsulfonsäuren, z. B. Methan- oder p-Toluolsulfonsäure, gebildet werden, oder Salze mit Basen, wie entsprechende Alkalimetall- oder Erdalkalimetallsalze, z. B. Natrium-, Kalium- oder Magnesiumsalze, pharmazeutisch verwendbare Uebergangsmetallsalze, wie Zink- oder Kupfersalze, oder Salze mit Ammoniak oder organischen Aminen, wie cyclischen Aminen, wie Mono-, Di- oder Triniederalkylaminen, wie Hydroxyniederalkylaminen, z. B. Mono-, Di- oder Trihydroxyniederalkylaminen, Hydroxyniederalkyl-niederalkylaminen oder Polyhydroxyniederalkylaminen. Cyclische Amine sind z. B. Morpholin, Thiomorpholin, Piperidin oder Pyryolidin. Als Mononiederalkylamine kommen beispielsweise Ethyl- und t-Butylamin, als Diniederalkylamine beispielsweise Diethyl- und Diisopropylamin und als Triniederalkylamine beispielsweise Trimethyl- und Triethylamin in Betracht. Entsprechende Hydroxyniederalkylamine sind z. B. Mono-, Di- und Triethanolamin; Hydroxyniederalkyl-niederalkyl-amine sind z.B. N,N-Dimethylamino- und N,N-Diethylamino-ethanol; als Polyhydroxyniederalkylamin kommt z. B. Glucosamin in Frage. Umfasst sind ferner für pharmazeutische Verwendungen ungeeignete Salze, da diese beispielsweise für die Isolierung bzw. Reinigung von freien Verbindungen I sowie von deren pharmazeutisch verwendbaren Salzen verwendet werden können.

Vor- und nachstehend sind unter mit "Nieder" bezeichneten Resten und Verbindungen, sofern nicht abweichend definiert, solche zu verstehen, die bis und mit 7, vor allem bis und mit 4, C-Atome aufweisen.

Geradkettiges $C_2$-$C_4$-Alk-1-en-1-yl ist Vinyl,(Z)-Propen-1-yl, (E)-Propen-1-yl, (Z)-But-1-en-1-yl oder (E)-But-1-en-1-yl.

Die Verbindungen I und ihre pharmazeutisch verwendbaren Salze weisen beispielsweise wertvolle pharmakologische Eigenschaften, insbesondere eine ausgeprägte antagonistische Wirkung gegenüber Leukotrienen, auf.

So hemmen sie z.B. in vitro in einem Konzentrationsbereich von etwa 0,001 bis etwa 1 µmol/l die durch Leukotrien $D_4$ ($LTD_4$) induzierte Kontraktion eines glatten Muskels. Diese als $LTD_4$-Antagonismus bezeichnete Wirkung kann experimentell z.B. verifiziert werden, indem in Segmenten, die dem Ileum eines 300 bis

400 g schweren Meerschweinchens entnommen und in einem Organbad in Tyrode-Lösung bei 38°C und unter Begasung mit einem Gemisch aus 95 % Sauerstoff und 5 % Kohlendioxid bei einer Belastung von 1 g inkubiert wurden, mit synthetischem Leukotrien $D_4$ (in Form des Kaliumsalzes) Kontraktionen ausgelöst und diese isotonisch registriert werden. Das Ausmaß der Hemmung der Kontraktionen durch die Prüfsubstanz wird nach einer Vorinkubation von 2 Minuten in Form der $IC_{50}$ ermittelt, wobei als $IC_{50}$ diejenige Konzentration bezeichnet wird, bei welcher die Testkontraktionen um 50 % reduziert sind.

Die Verbindungen I und ihre pharmazeutisch verwendbaren Salze sind auch in vivo ausgezeichnet wirksam. So kann z. B. im Bronchokonstriktions-Standardtest am Meerschweinchen bei Verabreichung einer Aerosollösung, enthaltend von etwa 0,00001 bis etwa 1 Gewichtsprozent Prüfsubstanz, ein deutlicher $LTD_4$-antagonistischer Effekt beobachtet werden. In diesem Testmodell anästhetisiert man männliche, 400 bis 700 g schwere, Meerschweinchen intraperitoneal mit 1,4 g/kg Urethan und führt eine Polyethylenkanüle in die Vena jugularis ein. Eine zweite Polyethylenkanüle wird in die Trachea eingeführt. Mittels einer in die Speiseröhre eingeführten Kanüle, welche mit einem Statham-Druck-Transduktor verbunden ist, wird der Druck in der Speiseröhre aufgezeichnet. Das Tier wird in eine luftdicht verschließbare Plexiglaskammer gelegt, die mit einer Fleisch'schen Röhre Nr. 000 und einem Validyne-Transducer MP 45-1 verbunden ist. Mit dieser Anordnung wird der Flow gemessen. Nach der chirurgischen Präparierung der Versuchstiere wartet man eine gewisse Zeit, damit sich die pulmonalen Funktionen stabilisieren können. Die Prüfsubstanz wird anschließend gemäss dem nachfolgenden Protokoll verabreicht. Die Versuchstiere werden während einer Minute einer einprozentigen (Gewicht/Volumen) Aerosollösung der Prüfsubstanz oder destilliertem Wasser (zu Kontrollzwecken) ausgesetzt. Für alle Testverbindungen, welche durch Inhalation verabreicht werden, verwendet man ein Monaghan-Ultraschall-Sprühgerät (Modell 670), dessen Partikelgrösse sich zwischen 1 und 8 Mikron, mit einem Hauptanteil von 3 Mikron, bewegt. Wäßrige Lösungen werden jeweils frisch hergestellt und mit einem on-stream drug vial in die Kammer des Sprühgeräts eingeführt. Der produzierte Sprühnebel wird den Versuchstieren über eine Glaskammer von 65 ml Inhalt, die mit einer Kanüle mit der Trachea verbunden wird, verabreicht. Nach Ablauf der Behandlungszeit wird mit einem zweiten Monaghan-Ultraschall-Sprühgerät (Modell 670) und über eine gleiche Glaskammer $LTD_4$ (0,3 µg/ml) während 2 Minuten verabreicht. Es wird die Abnahme der Compliance in der 3. Minute nach $LTD_4$-Applikation abgelesen, und zwar wird der Mittelwert von drei Tieren mit dem Mittelwert von drei Kontrolltieren verglichen und die prozentuale Hemmung der Compliance (% Hemmung) nach folgender Formel errechnet:

$$\% \text{ Hemmung} = 100 - \frac{(100 - \text{Compliance Präparat}) \cdot 100}{(100 - \text{Compliance Kontrolle})}$$

Werden unterschiedliche Wirkstoffkonzentrationen untersucht, so wird die prozentuale Hemmung für jede Konzentration aufgezeichnet, und zwar wird "log Konzentration" auf der Abszisse gegen "prozentuale Hemmung" auf der Ordinate aufgetragen. Die $IC_{50}$ wird dann durch lineare Regressionsanalyse ermittelt.

Die Verbindungen I und ihre pharmazeutisch verwendbaren Salze weisen weiterhin den spezifischen und therapeutisch sehr bedeutsamen Vorteil einer verhältnismäßig langen Wirkungsdauer auf.

Die Verbindungen I und ihre pharmazeutisch verwendbaren Salze können daher überall dort therapeutische Anwendung finden, wo die Wirkung der Leukotriene zu krankhaften Zuständen führt, und diese mildern oder beheben. Die Leukotriene spielen eine wichtige Rolle u.a. bei der Entstehung von allergischen und entzündlichen Prozessen. Demzufolge können die Verbindungen I und ihre pharmazeutisch verwendbaren Salze beispielsweise als Wirkstoffe in Antiallergika verwendet werden, welche z. B. zur Behandlung von allergischen Zuständen und Erkrankungen, wie insbesondere von Asthma, aber auch von Heufieber sowie von obstruktiven Lungehkrankheiten, einschließlich der zystischen Fibrose, eingesetzt werden. Ein Gegenstand der Erfindung ist somit die Verwendung der Verbindungen I und ihrer pharmazeutisch verwendbaren Salze zur Herstellung von entsprechenden Arzneimitteln. Dabei ist die gewerbsmäßige Herrichtung der Wirksubstanzen eingeschlossen.

Bevorzugt sind im Rahmen der Erfindung Verbindungen der Formel I, worin R Vinyl, (Z)-Propen-1-yl oder (E)-Propen-1-yl bedeutet, und ihre Salze.

Besonders bevorzugt sind im Rahmen der Erfindung Verbindungen der Formel I, worin R Vinyl oder (Z)-Propen-1-yl bedeutet, und ihre Salze.

Ganz besonders bevorzugt sind im Rahmen der Erfindung die Verbindung der Formel I, worin R Vinyl bedeutet, und ihre Salze.

Namentlich bevorzugt sind im Rahmen der Erfindung die in den Beispielen genannten Verbindungen der Formel I und ihre Salze.

Ebenfalls Gegenstand der vorliegenden Erfindung ist ein Verfahren zur Herstellung von Verbindungen der Formel I und ihrer Salze, z.B. dadurch gekennzeichnet, dass man

a) eine Verbindung der Formel

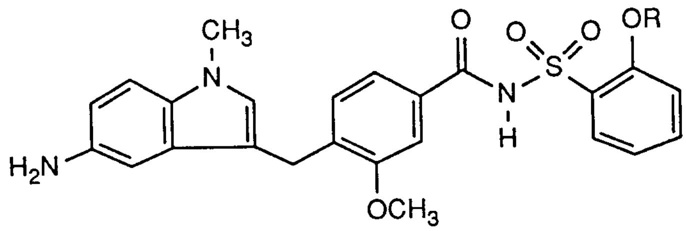

(II)

oder ein Salz davon mit einer Verbindung der Formel

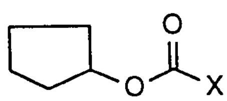

(III),

worin X eine nucleofuge Abgangsgruppe ist, unter Abspaltung einer Verbindung H-X umsetzt oder

b) in einer Verbindung der Formel

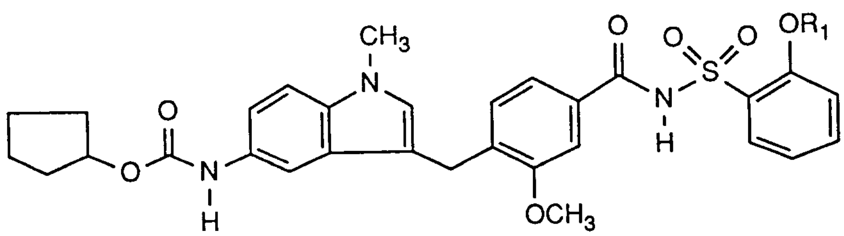

(IV),

worin $R_1$ eine in R überführbare Gruppe bedeutet, oder einem Salz davon $R_1$ in R überführt und gewünschtenfalls jeweils ein verfahrensgemäss erhältliches Isomerengemisch in die Komponenten auftrennt und/oder eine verfahrensgemäss erhältliche freie Verbindung I in ein Salz überführt oder ein verfahrensgemäss erhältliches Salz einer Verbindung I in die freie Verbindung I oder in ein anderes Salz umwandelt.

Die vor- und nachstehend beschriebenen Umsetzungen werden in an sich bekannter Weise durchgeführt, z.B. in Ab- oder üblicherweise in Anwesenheit eines geeigneten Lösungs- oder Verdünnungsmittels oder eines Gemisches derselben, wobei man je nach Bedarf unter Kühlen, bei Raumtemperatur oder unter Erwärmen, z.B. in einem Temperaturbereich von etwa -80 °C bis zur Siedetemperatur des Reaktionsmediums, vorzugsweise von etwa -20 ° bis etwa +150 °C, und, falls erforderlich, in einem geschlossenen Gefäss, unter Druck, in einer Inertgasatmosphäre und/oder unter wasserfreien Bedingungen arbeitet. Besonders vorteilhafte Reaktionsbedingungen können den Beispielen entnommen werden.

Das vor- und nachstehend aufgeführte Ausgangsmaterial, das für die Herstellung der Verbindungen I und ihrer Salze verwendet wird, ist bekannt oder kann nach an sich bekannten Methoden, z.B. gemäss den nachstehend beschriebenen Verfahrensweisen, hergestellt werden.

Für Salze von vor- und nachstehend aufgeführten Ausgangsmaterialien gilt das vorstehend für Salze von Verbindungen I Gesagte in analoger Weise.

Variante a):

Nucleofuge Abgangsgruppen X in Verbindungen III sind beispielsweise gegebenenfalls veretherte oder veresterte Hydroxy- oder Mercaptogruppen, Sulfinyl- und Sulfonylgruppen, ferner Sulfoniumgruppen. Verethertes Hydroxy ist z. B. Niederalkoxy oder gegebenenfalls substituiertes Phenylniederalkoxy. Verestertes Hydroxy ist insbesondere mit einer Mineralsäure oder einer organischen Sulfonsäure verestertes Hydroxy,

vor allem Halogen, Sulfonyloxy, ferner Niederalkanoyloxy. Verethertes Mercapto ist z. B. Niederalkylthio, gegebenenfalls substituiertes Arylthio oder gegebenenfalls substituiertes Arylniederalkylthio. Verestertes Mercapto ist z.B. Niederalkanoylthio. Sulfinyl ist z. B. Niederalkansulfinyl, gegebenenfalls substituiertes Arylsulfinyl oder gegebenenfalls substituiertes Benzylsulfinyl. Sulfonyl ist z. B. Niederalkansulfonyl, gegebenenfalls substituiertes Arylsulfonyl oder gegebenenfalls substituiertes Benzylsulfonyl. Sulfoniumgruppen sind z. B. Diniederalkylsulfoniumgruppen.

Die Umsetzung einer Verbindung II oder eines Salzes davon mit einer Verbindung III erfolgt in üblicher Weise, z. B. unter Kühlen, bei Raumtemperatur oder unter Erwärmen, gegebenenfalls in Gegenwart eines inerten Lösungs- oder Verdünnungsmittels oder eines Gemisches derselben, gegebenenfalls in Gegenwart eines basischen Mittels und/oder unter Inertgas.

Als inerte Lösungs- oder Verdünnungsmittel sind beispielsweise cyclische Ether, aromatische Kohlenwasserstoffe, N,N-Diniederalkylniederalkansäureamide, Phosphorsäureniederalkylamide, Diniederalkylsulfoxide, cyclische Amine, Niederalkanole und insbesondere gegebenenfalls halogenierte Kohlenwasserstoffe zu nennen, beispielsweise Tetrahydrofuran, Dioxan, Benzol, Toluol, Xylol, N,N-Dimethylformamid, Hexamethylphosphorsäuretriamid, Dimethylsulfoxid, Pyridin, N-Methylmorpholin, Methanol, Ethanol und insbesondere Hexan und Di- und Trichlormethan.

Geeignete basische Mittel sind z.B. Alkalimetall- oder Erdalkalimetall-hydroxide, -hydride, -amide, -niederalkanolate, -carbonate, -diniederalkylamide oder -niederalkylsilylamide, Niederalkylamine, gegebenenfalls N-niederalkylierte Cycloalkylamine, basische Heterocyclen, Ammoniumhydroxide sowie carbocyclische Amine. Beispielhaft seien Natrium-hydroxid, -hydrid, -amid, -methanolat, Kalium-tert.-butanolat, -carbonat, Lithiumdiisopropylamid, Kalium-bis(trimethylsilyl)-amid, Calciumhydrid, Triethylamin, Cyclohexylamin, N-Cyclohexyl-N,N-dimethyl-amin, Pyridin, N-Methylmorpholin, Benzyl-trimethyl-ammoniumhydroxid sowie 1,5-Diazabicyclo[5.4.0]undec-5-en (DBU) genannt.

In einer bevorzugten Ausführungsform der Variante a) wird eine Verbindung II bei Raumtemperatur, unter Stickstoff oder Argon, in einem halogenierten Kohlenwasserstoff, vorzugsweise in Dichlormethan, und in Gegenwart eines basischen Heterocyclus, vorzugsweise in Gegenwart von N-Methylmorpholin, mit einer Verbindung III, worin X Halogen, vorzugsweise Chlor, ist, umgesetzt.

Die Verbindungen II und deren Salze lassen sich z.B. aus den entsprechenden 5-Nitroindolverbindungen durch Reduktion der Nitrogruppe zur Aminogruppe herstellen. Diese 5-Nitroindole wiederum sind z. B. durch Umsetzung von entsprechenden Verbindungen, die anstelle der RO-Benzolsulfonylaminocarbonylgruppe eine Carboxylgruppe tragen, mit entsprechenden, in 2-Position durch eine RO-Gruppe substituierten, Benzolsulfonsäureamiden zugänglich. Die Carboxylverbindungen ihrerseits lassen sich z. B. durch Hydrolyse entsprechender Carbonsäure-derivate, z. B. -ester, erhalten, während die 2-(RO)-Benzolsulfonsäureamide z. B. durch Eliminierung von H-X aus Vorstufen, die anstelle der in dem Rest R enthaltenen Doppelbindung ein Wasserstoffatom und eine nucleofuge Abgangsgruppe X, z. B. der vorstehend erwähnten Art, vorzugsweise Halogen, wie Chlor, enthalten, erhältlich sind. In den Beispielen finden sich nähere Angaben zu derartigen Verfahren für die Herstellung der Ausgangsverbindungen II und ihrer Vorstufen.

Die Verbindungen III sind bekannt oder können in Analogie zu den bekannten Verbindungen hergestellt werden.

<u>Variante b):</u>

Geeignete Gruppen $R_1$ sind z. B. solche, die anstelle der in dem jeweiligen Rest R enthaltenen Doppelbindung ein Wasserstoffatom und eine nucleofuge Abgangsgruppe X enthalten, ansonsten aber mit dem Rest R identisch sind.

Nucleofuge Abgangsgruppen X in Gruppen $R_1$ sind beispielsweise gegebenenfalls veretherte oder veresterte Hydroxy- oder Mercaptogruppen, Sulfinyl- und Sulfonylgruppen, ferner Sulfoniumgruppen. Verethertes Hydroxy ist z. B. Niederalkoxy oder gegebenenfalls substituiertes Phenylniederalkoxy. Verestertes Hydroxy ist insbesondere mit einer Mineralsäure oder einer organischen Sulfonsäure verestertes Hydroxy, vor allem Halogen, Sulfonyloxy, ferner Niederalkanoyloxy. Verethertes Mercapto ist z. B. Niederalkylthio, gegebenenfalls substituiertes Arylthio oder gegebenenfalls substituiertes Arylniederalkylthio. Verestertes Mercapto ist z. B. Niederalkanoylthio. Sulfinyl ist z. B. Niederalkansulfinyl, gegebenenfalls substituiertes Arylsulfinyl oder gegebenenfalls substituiertes Benzylsulfinyl. Sulfonyl ist z. B. Niederalkansulfonyl, gegebenenfalls substituiertes Arylsulfonyl oder gegebenenfalls substituiertes Benzylsulfonyl. Sulfoniumgruppen sind z. B. Diniederalkylsulfoniumgruppen.

Die Ueberführung von $R_1$ in R in einer Verbindung IV oder einem Salz davon erfolgt in üblicher Weise, z. B. unter Kühlen, bei Raumtemperatur oder unter Erwärmen, gegebenenfalls in Gegenwart eines inerten Lösungs- oder Verdünnungsmittels oder eines Gemisches derselben, gegebenenfalls in Gegenwart eines

basischen Mittels und/oder unter Inertgas.

Als inerte Lösungs- oder Verdünnungsmittel sind beispielsweise cyclische Ether, aromatische Kohlenwasserstoffe, N,N-Diniederalkylniederalkansäureamide, Phosphorsäureniederalkylamide, Diniederalkylsulfoxide, cyclische Amine, gegebenenfalls halogenierte Kohlenwasserstoffe und insbesondere Niederalkanole zu nennen, beispielsweise Tetrahydrofuran, Dioxan, Benzol, Toluol, Xylol, N,N-Dimethylformamid, Hexamethylphosphorsäuretriamid, Dimethylsulfoxid, Pyridin, N-Methylmorpholin, Hexan, Di- und Trichlormethan und insbesondere Methanol, Ethanol und tert.-Butanol.

Geeignete basische Mittel sind z. B. Alkalimetall- oder Erdalkalimetall-hydroxide, -hydride, -amide, -niederalkanolate, -carbonate, -diniederalkylamide oder -niederalkylsilylamide, Niederalkylamine, gegebenenfalls N-niedeialkylierte Cycloalkylamine, basische Heterocyclen, Ammoniumhydroxide sowie carbocyclische Amine. Beispielhaft seien Natrium-hydroxid, -hydrid, -amid, -methanolat, Kalium-tert.-butanolat, -carbonat, Lithiumdiisopropylamid, Kalium-bis(trimethylsilyl)-amid, Calciumhydrid, Triethylamin, Cyclohexylamin, N-Cyclohexyl-N,N-dimethyl-amin, Pyridin, N-Methylmorpholin, Benzyl-trimethyl-ammoniumhydroxid sowie 1,5-Diazabicyclo[5.4.0]undec-5-en (DBU) genannt.

In einer bevorzugten Ausführungsform der Variante b) wird eine Verbindung IV in einem Temperaturbereich von etwa 0° bis etwa +100°C in einem Niederalkanol, vorzugsweise in tert.-Butanol, mit einem Alkalimetallniederalkanolat, vorzugsweise mit Kalium-tert.-butanolat, umgesetzt.

Die Verbindungen IV und deren Salze lassen sich z. B. dadurch erhalten, daß man eine 5-Aminoindolverbindung, die einer Verbindung der Formel II entspricht, aber anstelle der Gruppe R einen Rest $R_1$ trägt, mit einer Verbindung der Formel III umsetzt. Die 5-Aminoindole lassen sich z. B. aus den entsprechenden 5-Nitroindolverbindungen durch Reduktion der Nitrogruppe zur Aminogruppe herstellen. Diese 5-Nitroindole wiederum sind z. B. durch Umsetzung von entsprechenden Verbindungen, die anstelle der $R_1$O-Benzolsulfonylaminocarbonylgruppe eine Carboxylgruppe tragen, mit entsprechenden, in 2-Position durch eine $R_1$O-Gruppe substituierten, Benzolsulfonsäureamiden zugänglich. Die Carboxylverbindungen ihrerseits lassen sich z. B. durch Hydrolyse entsprechender Carbonsäure-derivate, z. B. -ester, erhalten, während die 2-($R_1$O)-Benzolsulfonsäureamide bekannt sind oder in Analogie zu den bekannten Verbindungen hergestellt werden können. In den Beispielen finden sich nähere Angaben zu derartigen Verfahren für die Herstellung der Ausgangsverbindungen IV und ihrer Vorstufen.

Salze von Verbindungen I können in an sich bekannter Weise hergestellt werden. So erhält man beispielsweise Säureadditionssalze von Verbindungen I durch Behandeln mit einer geeigneten Säure oder einem geeigneten Ionenaustauscherreagens und Salze mit Basen durch Behandeln mit einer geeigneten Base oder einem geeigneten Ionenaustauscherreagens. Salze von Verbindungen I können in üblicher Weise in die freien Verbindungen I überführt werden, Säureadditionssalze z.B. durch Behandeln mit einem geeigneten basischen Mittel oder einem geeigneten Ionenaustauscherreagens und Salze mit Basen z. B. durch Behandeln mit einer geeigneten Säure oder einem geeigneten Ionenaustauscherreagens.

Salze von Verbindungen I können in an sich bekannter Weise in andere Salze von Verbindungen I umgewandelt werden, Säureadditionssalze beispielsweise in andere Säureadditionssalze, z.B. durch Behandeln eines Salzes einer anorganischen Säure, wie eines Hydrochlorids, mit einem geeigneten Metallsalz, wie einem Natrium-, Barium- oder Silbersalz, einer Säure, z.B. mit Silberacetat, in einem geeigneten Lösungsmittel, in welchem ein sich bildendes anorganisches Salz, z.B. Silberchlorid, unlöslich ist und damit aus dem Reaktionsgemisch ausscheidet.

Je nach Verfahrensweise bzw. Reaktionsbedingungen können die Verbindungen I mit salzbildenden Eigenschaften in freier Form oder in Form von Salzen erhalten werden.

Infolge der engen Beziehung zwischen der Verbindung I in freier Form und in Form ihrer Salze sind im vorausgegangenen und nachfolgend unter der freien Verbindung I bzw. ihren Salzen sinn- und zweckgemäss gegebenenfalls auch die entsprechenden Salze bzw. die freie Verbindung I zu verstehen.

Die Verbindungen I einschliesslich ihrer Salze von salzbildenden Verbindungen können auch in Form ihrer Hydrate erhalten werden und/oder andere, beispielsweise gegebenenfalls zur Kristallisation von in fester Form vorliegenden Verbindungen verwendete, Lösungsmittel einschliessen.

Die Verbindungen I und ihre Salze können, je nach Wahl der Ausgangsstoffe und Arbeitsweisen, in Form eines der möglichen Isomeren oder als Gemisch derselben vorliegen. Dabei sind als reine Isomere z.B. reine Diastereomere, wie reine cis/trans-Isomere, erhältlich. Entsprechend können als Isomerengemische z.B. Diastereomerengemische vorliegen. Verfahrensgemäss oder anderweitig erhältliche Isomerengemische von Verbindungen I in freier Form oder in Salzform können in üblicher Weise in die Komponenten aufgetrennt werden, z. B. aufgrund der physikalisch-chemischen Unterschiede der Bestandteile in bekannter Weise durch fraktionierte Kristallisation, Destillation und/oder Chromatographie. Vorteilhaft isoliert man das wirksamere Isomere.

Die Erfindung betrifft auch diejenigen Ausführungsformen des Verfahrens, nach denen man von einer auf irgendeiner Stufe des Verfahrens als Zwischenprodukt erhältiichen Verbindung ausgeht und die fehlenden Schritte durchführt oder einen Ausgangsstoff in Form eines Derivates bzw. Salzes verwendet oder insbesondere unter den Reaktionsbedingungen bildet.

Beim Verfahren der vorliegenden Erfindung werden vorzugsweise solche Ausgangsstoffe und Zwischenprodukte, jeweils in freier Form oder in Salzform, verwendet, welche zu den eingangs als besonders wertvoll geschilderten Verbindungen I bzw. deren Salzen führen.

Neue Ausgangsstoffe und Zwischenprodukte, jeweils in freier Form oder in Salzform, für die Herstellung der Verbindungen I bzw. ihrer Salze, ihre Verwendung und Verfahren zu ihrer Herstellung bilden ebenfalls einen Gegenstand der Erfindung, wobei die Variable R die für die Verbindungen I angegebene Bedeutung hat.

Die Erfindung betrifft ebenfalls die Verwendung der Verbindungen I und ihrer pharmazeutisch verwendbaren Salze zur Behandlung von allergischen Zuständen und Erkrankungen,vorzugsweise in Form von pharmazeutisch verwendbaren Zubereitungen, insbesondere in einem Verfahren zur therapeutischen Behandlung des tierischen oder menschlichen Körpers, sowie ein solches Behandlungsverfahren.

Die Erfindung betrifft gleichfalls pharmazeutische Präparate, die eine Verbindung I oder ein pharmazeutisch verwendbares Salz davon als Wirkstoff enthalten, sowie Verfahren zu ihrer Herstellung. Bei diesen pharmazeutischen Präparaten handelt es sich um solche zur enteralen, wie oralen, ferner rektalen, Verabreichung, um solche zur parenteralen Verabreichung, um solche zur lokalen Verabreichung und vor allem um solche zur Inhalationsverabreichung an Warmblüter, vor allem an Menschen, wobei der pharmakologische Wirkstoff allein oder zusammen mit üblichen pharmazeutischen Hilfsstoffen enthalten ist. Die pharmazeutischen Präparate enthalten (in Gewichtsprozenten) z.B. von etwa 0,001 % bis 100 %, vorzugsweise von etwa 0,1 % bis etwa 50 %, des Wirkstoffs.

Pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren, hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert und das Gemisch bzw. Granulat, wenn erwünscht oder notwendig nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Tragaganth, Methylcellulose und/oder Polyvinylpyrrolidon, und, wenn erwünscht, Sprengmittel, wie die obengenannten Stärken, ferner Carboxymethylstärke, quervernetztes Polyvinylpyrrolidon, Agar oder Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaft-resistenten, Ueberzügen versehen, wobei man u.a. konzentrierte Zuckerlösungen, welche gegebenfalls arabischen Gummi, Talk, Polyvinylpyrrolidon, Polyäthylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat oder Triglyceride, verwendet, oder wässrige Injektionssuspensionen, welche viskositätserhöhende Stoffe, z.B. Natriumcarboxymethylcellulose, Sorbit und/oder Dextran, und gegebenenfalls auch Stabilisatoren enthalten.

Pharmazeutische Präparate zur lokalen Verabreichung sind z. B. für die topische Behandlung der Haut Lotionen, Cremes und Salben, d. h. flüssige oder semifeste Oel-in-Wasser- oder Wasser-in-Oel-Emulsionen, Fettsalben, die wasserfrei sind, Pasten, d. h. Crèmes und Salben mit sekretabsorbierenden Puderbestandteilen, Gele, die wässrig, wasserarm oder wasserfrei sind und aus quellbaren, gelbildenden Materialien bestehen, Schäume, d. h. in Aerosolform vorliegende flüssige Oel-in-Wasser-Emulsionen, welche aus Druckbehältern verabreicht werden, und eine wässrig-ethanolische Grundlage aufweisende Tinkturen, welche jeweils weitere übliche pharmazeutische Hilfsstoffe, wie Konservierungsmittel, enthalten können. Für die lokale Behandlung der Augen eignen sich z. B. Augentropfen, welche den Wirkstoff in steriler wässriger oder öliger Lösung enthalten, und Augensalben, die vorzugsweise ebenfalls in steriler Form hergestellt werden. Für die lokale Behandlung der Nase sind z. B. Sprays, ähnlich den weiter unten beschriebenen für die Behandlung der Atemwege, grobe Puder, die durch schnelles Inhalieren durch die Nasenlöcher verabreicht werden, und vor allem Nasentropfen, welche den Wirkstoff in wässriger oder öliger Lösung enthalten, geeignet. Für die lokale Behandlung der Mundhöhle eignen sich z. B. Lutschbonbons und Pastillen, welche den Wirkstoff in einer z. B. aus Zucker und Gummiarabikum oder Tragantgummi gebildeten inerten Masse enthalten, welcher Geschmacksstoffe beigegeben sein können. Die Herstellung der pharmazeutischen Präparate zur lokalen Verabreichung erfolgt in an sich bekannter Weise durch Vermischung des Wirkstoffs mit den pharmazeutischen Hilfsstoffen; z. B. durch Lösen oder Suspendieren des Wirkstoffs in der Grundlage oder in einem Teil davon, falls notwendig. Zur Herstellung von Emulsionen, in denen der Wirkstoff in einer der flüssigen Phasen gelöst ist, wird der Wirkstoff in der Regel vor der Emulgierung in dieser gelöst; zur Herstellung von Suspensionen, in denen der Wirkstoff in der Emulsion suspendiert ist, wird der Wirkstoff nach der Emulgierung mit einem Teil der Grundlage vermischt und dann dem Rest der Formulierung beigegeben.

Pharmazeutische Präparate zur Inhalationsverabreichung sind solche, in denen der Wirkstoff in mikronisierter Form vorliegt, d. h. in denen die Partikelgrösse des Wirkstoffs weniger als 20 µm, insbesondere weniger als 10 µm und vorteilhaft weniger als 5 µm, beträgt, z. B. mikronisierte Pulver und Aerosole, die in Form von Sprays verabreicht werden. Die mikronisierten Pulver enthalten den Wirkstoff allein oder zusammen mit einem inerten Trägerstoff, wie Lactose, vorteilhaft zusammen mit einem der nachstehend erwähnten Treibmittel. Aerosole sind Lösungen, Suspensionen oder Emulsionen des Wirkstoffs in einer geeigneten, pharmazeutisch verwendbaren, flüssigen Phase, wie in Ethanol oder Wasser oder einem entsprechenden Gemisch, können nach Bedarf auch andere pharmazeutische Hilfsstoffe, wie nicht-ionische oder anionische oberflächenaktive Mittel, Emulgatoren und Stabilisatoren, und/oder Wirkstoffe anderer Art aufweisen und enthalten ein Treibmittel, z. B. ein inertes Gas, wie Butan, unter erhöhtem Druck oder insbesondere eine leicht flüchtige, vorzugsweise unter normalem Druck unterhalb der üblichen Raumtemperatur (z.B. zwischen etwa -30°C und etwa +10°C) siedende, Flüssigkeit, wie ein mindestens teilweise fluoriertes polyhalogeniertes Niederalkan, oder ein Gemisch solcher Flüssigkeiten. Zur Herstellung der pharmazeutischen Präparate in zur Inhalationsverabreichung fertiger Form wird eine entsprechende pharmazeutische Zusammensetzung zusammen mit dem Treibmittel in geeignete Behälter, wie Flacons oder Druckflaschen, abgefüllt, welche mit einer geeigneten Versprühungseinrichtung, z. B. einem Ventil, versehen sind. Das Ventil ist vorzugsweise als Dosierungsventil konstruiert, welches bei Betätigung eine vorbestimmte Menge des Behälterinhalts, entsprechend einer vorbestimmten Dosis des Wirkstoffs, freigibt. Bei der Herstellung der fertigen Arzneimittelform kann man auch so vorgehen, dass entsprechende Mengen der pharmazeutischen Zusammensetzung und des Treibmittels separat in die Behälter abgefüllt werden und erst dort zur Vermischung kommen.

Die Dosierung des Wirkstoffs kann von verschiedenen Faktoren, wie Wirksamkeit und Wirkungsdauer des Wirkstoffs, Stärke der zu behandelnden Krankheit bzw. ihrer Symptome, Applikationsweise, Warmblüter-Spezies, -Geschlecht, -Alter, -Gewicht und/oder individuellem Zustand des Warmblüters, abhängen. Im Normalfall ist für einen etwa 75 kg schweren Warmblüter eine ungefähre Tagesdosis von etwa 10 mg bis etwa 1500 mg, insbesondere von etwa 25 bis etwa 250 mg, gegebenenfalls in mehreren, gegebenenfalls gleichen, Teildosen, zu veranschlagen.

Die nachfolgenden Beispiele illustrieren die vorstehend beschriebene Erfindung, ohne sie in ihrem Umfang in irgendeiner Weise einzuschränken. Temperaturen sind in Celsiusgraden angegeben. "DMSO" steht für "Dimethylsulfoxid".

Beispiel 1: Zu einer Lösung von 3,99 g 2-Vinyloxybenzolsulfonsäure-N-[4-(5-amino-1-methyl-indol-3-ylmethyl)-3-methoxy-benzoyl]amid in 30 ml Dichlormethan und 2,3 g N-Methylmorpholin setzt man eine Lösung von 1,15 g Chlorameisensäurecyclopentylester in 20 ml Dichlormethan zu. Die Mischung wird zwei Stunden unter Einleiten von Stickstoff bei Raumtemperatur gerührt und dann auf ein Gemisch von 40 g Eis und 20 ml 1 N-Salzsäure gegossen. Die organische Phase wird abgetrennt, nacheinander mit 20 ml gesättigter Kochsalzlösung und 20 ml Wasser gewaschen, über Magnesiumsulfat getrocknet und unter 11 Torr bei 40° eingedampft. Den Rückstand chromatographiert man mittels MPLC an 300 g Kieselgel (Merck, Lichroprep Si 60/25-40 µm). Die Fraktionen 1-4, eluiert mit Dichlormethan, werden verworfen. Die Fraktionen 5-12, eluiert mit Dichlormethan, werden vereinigt und unter 11 Torr bei 40° eingedampft. Den Rückstand löst man in circa 10 ml Dichlormethan und setzt zur Lösung Petrolether bis zur beginnenden Trübung zu. Die ausgeschiedenen Kristalle werden abfiltriert, mit Dichlormethan/Petrolether (1:1) gewaschen und unter 0,01 Torr bei 60° getrocknet. Das 2-Vinyloxybenzolsulfonsäure-N-[4-(5-cyclopentyloxycarbonylamino-1-methyl-indol-3-ylmethyl)-3-methoxy-benzoyl]amid (also die Verbindung der Formel I, worin R Vinyl ist) schmilzt bei 161-165°.

Das Ausgangsmaterial kann z. B. wie folgt hergestellt werden:

a) 10,0 g 3-Methoxy-4-(1-methyl-5-nitro-indol-3-ylmethyl)-benzoesäuremethylester werden in 240 ml Tetrahydrofuran und 180 ml Methanol gelöst. Die Lösung wird mit einer Lösung von 4,2 g Lithiumhydroxid (Monohydrat) in 70 ml Wasser versetzt. Die Mischung wird 15 Stunden bei Raumtemperatur gerührt und unter 11 Torr bei 50° auf ein Volumen von circa 50 ml eingeengt. Man verdünnt den Rückstand mit 50 ml Wasser und stellt das Gemisch mit 1 N-Salzsäure sauer. Die ausgeschiedenen gelben Kristalle werden abfiltriert, mit 20 ml Wasser gewaschen und unter 0,01 Torr bei 40° 20 Stunden getrocknet. Die 3-Methoxy-4-(1-methyl-5-nitro-indol-3-ylmethyl)-benzoesäure schmilzt bei 263-265°.

b) Eine Mischung von 2,72 g N-(3-Dimethylaminopropyl)-N'-ethyl-carbodiimid-hydrochlorid, 2,72 g 2-Vinyloxybenzolsulfonsäureamid und 1,72 g 4-Dimethylaminopyridin in 120 ml Dichlormethan wird unter Rühren und Einleiten von Argon bei Raumtemperatur mit einer Lösung von 4,0 g 3-Methoxy-4-(1-methyl-5-nitro-indol-3-ylmethyl)-benzoesäure in 200 ml Tetrahydrofuran versetzt. Die Mischung wird 5 Stunden bei 42° und anschliessend 15 Stunden bei Raumtemperatur gerührt. Die ausgeschiedenen gelben Kristalle werden abfiltriert und in 300 ml Trichlormethan/Methanol (9:1) gelöst und die Lösung an 600 g Silicagel chromatographiert. Die Fraktionen 1-5, eluiert mit je 300 ml Trichlormethan/Methanol (9:1), werden vereinigt und unter 11 Torr bei 40° eingedampft. Den Rückstand verrührt man mit Diethylether, wobei das 2-Vinyloxybenzolsulfonsäure-N-[4-(1-methy-5-nitro-indol-3-ylmethyl)-3-methoxy-benzoyl]amid auskristallisiert (gelbe Kristalle; Smp.: 165-170°).

c) Eine Lösung von 2,0 g 2-Vinyloxybenzolsulfonsäure-N-[4-(1-methyl-5-nitro-indol-3-ylmethyl)-3-methoxy-benzoyl]amid in 40 ml Dimethylsulfoxid wird nach Zusatz von 1,0 g Lindlar-Katalysator-[Palladium auf Calciumcarbonat (5 %), mit Blei vergiftet] und 10 mg 1,8-Dihydroxy-3,6-dithia-oktan 20 Stunden bei 20-22° unter Normaldruck hydriert. Zur Abtrennung des Katalysators wird das Gemisch durch Hyflo Super Cel filtriert. Das Filtrat wird unter 0,01 Torr bei 30° eingedampft. Den Rückstand chromatographiert man an 300 g Silicagel. Die Fraktionen 1-3, eluiert mit je 100 ml Ethylacetat, werden verworfen. Die Fraktionen 4-15, eluiert mit je 100 ml Ethylacetat/Methanol (9:1), werden vereinigt und unter 11 Torr bei 40° eingedampft. Den Rückstand verrührt man mit Diethylether, wobei das 2-Vinyloxybenzolsulfonsäure-N-[4-(5-amino-1-methyl-indol-3-ylmethyl)-3-methoxy-benzoyl]amid auskristallisiert [rosafarbene Kristalle, Smp.: 145-150°, $^1$H-NMR (400 MHz, DMSO-$d_6$): 7,87 (d, d, 1H), 7,49 (m, 2H), 7,34 (d, d, 1H), 7,16 (t, 1H), 7,09 (m, 2H), 6,95 (d, 1H), 6,88 (s, 1H), 6,71 (d, d, 1H), 6,64 (d, 1H), 6,57 (d, d, 1H), 4,67 (d, d, 1H), 4,42 (d, d, 1H), 3,84 (s, 5H), 3,61 (s, 3H)].

d) Eine Suspension von 11,8 g 2-(2-Chlorethoxy)benzolsulfonsäureamid in 150 ml tert.-Butanol wird bei Raumtemperatur unter Rühren portionsweise mit 23,14 g Kalium-tert.-butanolat versetzt. Die Mischung wird 4 Stunden bei 80° gerührt, abgekühlt und unter 11 Torr bei 40° eingedampft. Man löst den Rückstand in circa 100 ml Eiswasser, stellt die Lösung mit konzentrierter Salzsäure auf pH 7, filtriert die weissen Kristalle ab und trocknet sie 15 Stunden unter 11 Torr bei 70°. Das 2-Vinyloxybenzolsulfonsäureamidschmilzt bei 140-142°.

Beispiel 2: Eine Suspension von 1,0 g 2-(2-Chlorethoxy)benzolsulfonsäure-N-[4-(5-cyclopentyloxycarbonylamino-1-methyl-indol-3-ylmethyl)-3-methoxy-benzoyl]amid in 4,7 ml tert.-Butanol wird bei Raumtemperatur unter Rühren mit 0,72 g Kalium-tert.-butanolat versetzt. Die Mischung wird 1 Stunde bei 80° gerührt und auf eine Mischung von 30 ml Eiswasser und 3 ml 2 N-Salzsäure gegossen. Die Suspension wird zweimal mit je 50 ml Ethylacetat extrahiert. Die vereinigten organischen Phasen werden nacheinander mit 20 ml gesättigter Natriumhydrogencarbonatlösung und zweimal mit je 20 ml Sole gewaschen, über Magnesiumsulfat getrocknet und unter 11 Torr bei 40° eingedampft. Den Rückstand chromatographiert man mittels MPLC an 80 g Kieselgel (Merck, Lichroprep Si 60/25-40 µm). Die Fraktionen 1-5, eluiert mit Dichlormethan, werden verworfen. Die Fraktionen 6-12, eluiert mit Dichlormethan, werden vereinigt und unter 11 Ton bei 40° eingedampft. Den Rückstand kristallisiert man aus Dichlormethan/Petrolether. Das 2-Vinyloxybenzolsulfonsäure-N-[4-(5-cyclopentyloxycarbonylamino-1-methyl-indol-3-ylmethyl)-3-methoxy-benzoyl]amid schmilzt bei 161-165°.

Das Ausgangsmaterial kann z. B. wie folgt hergestellt werden:

a) Analog Beispiel 1b) erhält man das 2-(2-Chlorethoxy)benzolsulfonsäure-N-[4-(1-methyl-5-nitro-indol-3-ylmethyl)-3-methoxy-benzoyl]amid [gelbes Pulver, Smp.: 150-160° (nach Verreiben mit Diethylether), $^1$H-NMR (360 MHz, DMSO-$d_6$): 8,48 (d, 1H), 8,00 (d, d, 1H), 7,90 (d, d, 1H), 7,59 (d, 1H), 7,54 (d, 1H), 7,46 (d, d, 1H), 7,43 (t, d, 1H), 7,26 (s, 1H), 7,15 (d, 1H), 7,06 (t, d, 1H), 4,29 (t, 2H), 4,08 (s, 2H), 3,89 (s, 3H), 3,80 (m, 5H)].

b) Eine Lösung von 1,05 g 2-(2-Chlorethoxy)benzolsulfonsäure-N-[4-(1-methyl-5-nitroindol-3-ylmethyl)-3-methoxy-benzoyl]amid in 45 ml Tetrahydrofuran wird nach Zusatz von 0,3 g Rhodium/C-Katalysator (5 %) 20 Stunden bei 20-22° unter Normaldruck hydriert, wobei nach 10 Stunden Hydrierdauer eine Zugabe von weiteren 0,1 g Rhodium/C-Katalysator (5 %) erfolgt. Zur Abtrennung des Katalysators wird das Gemisch durch Hyflo Super Cel filtriert. Das Filtrat wird unter 11 Torr bei 40° zur Trockne eingedampft. Der Rückstand wird an 35 g Silicagel flash-chromatographiert. Die Fraktionen 1-9, eluiert mit je 50 ml Ethylacetat, werden verworfen. Die Fraktionen 10-13, eluiert mit je 50 ml Ethylacetat, werden vereinigt und unter 11 Torr bei 40° eingedampft. Den Rückstand kristallisiert man aus Ethylacetat/Diethylether. Das 2-(2-Chlorethoxy)-benzolsulfonsäure-N-[4-(5-amino-1-methyl-indol-3-ylmethyl)-3-methoxy-benzoyl]amid (beigefarbene Kristalle) schmilzt bei 300° unter Zersetzung [$^1$H-NMR (400 MHz, DMSO-$d_6$): 7,85 (d, d, 1H), 7,53 (d, 1H), 7,46 (t, 1H), 7,38 (d, d, 1H), 7,10 (m, 3H), 6,98 (d, 1H), 6,83 (s, 1H), 6,60 (d, 1H), 6,54 (d, d, 1H), 4,26 (t, 2H), 3,86 (s, 3H), 3,85 (s, 2H) 3,82 (t, 2H), 3,60 (s, 3H)].

c) Analog Beispiel 1 erhält man das 2-(2-Chlorethoxy)benzolsulfonsäure-N-[4-(5-cyclopentyloxycarbonylamino-1-methyl-indol-3-ylmethyl)-3-methoxy-benzoyl]amid [rosafarbene Kristalle, Smp.: 168-171° (aus Dichlormethan/Petrolether),$^1$H-NMR (400 MHz, $CDCl_3$): 9,04 (s, 1H), 8,21 (d, d, 1H), 7,60 (m, 1H), 7,52 (m, 1H), 7,35 (d, 1H), 7,27 (d, d, 1H), 7,20 (m, 3H), 7,12 (d, 1H), 6,95 (d, 1H), 6,77 (s, 1H), 6,52 (s, 1H), 5,20 (m, 1H), 4,31 (t, 2H), 4,05 (s, 2H) 3,87 (s, 3H), 3,85 (t, 2H), 3,72 (s, 3H)].

Beispiel 3: Zu einer Lösung von 2,98 g (Z)-2-Propen-1-yloxybenzolsulfonsäure-N-[4-(5-amino-1-methyl-indol-3-ylmethyl)-3-methoxy-benzoyl]amid in 80 ml Dichlormethan und 1,79 g N-Methylmorpholin setzt man eine Lösung von 0,9 g Chlorameisensäurecyclopentylester in 15 ml Dichlormethan zu. Die Mischung wird zwei Stunden unter Argonatmosphäre bei Raumtemperatur gerührt und dann auf ein Gemisch von 30 g Eis und 30 ml 1 N-Salzsäure gegossen. Die organische Phase wird abgetrennt, nacheinander mit 20 ml gesättigter Kochsalzlösung und 20 ml Wasser gewaschen, über Magnesiumsulfat getrocknet und unter 11 Torr bei 40° eingedampft. Den Rückstand chromatographiert man mittels MPLC an 250g Kieselgel (Merck, Lichroprep Si 60/25-40 µm). Die Fraktionen 1-3, eluiert mit Dichlormethan, werden verworfen. Die Fraktionen 4-6, eluiert mit Dichlormethan, werden vereinigt und unter 11 Torr bei 40° eingedampft. Man verrührt den Rückstand mit Diethylether/Petrolether und filtriert die weissen Kristalle ab. Das (Z)-2-Propen-1-yloxybenzolsulfonsäure-N-[4-(5-cyclopentyloxycarbonylamino-1-methyl-indol-3-ylmethyl)-3-methoxy-benzoyl]amid schmilzt bei 181-183° [$^1$H-NMR (400 MHz, $CDCl_3$): 8,19 (d, d, 1H), 7,55 (t, d, 1H), 7,52 (m, 1H), 7,34 (d, 1H), 7,28-7,08 (m, 5H), 7,04 (d, d, 1H), 6,76 (s, 1H), 6,30 [d, q, 1H; $^3J_{(OCH=CH)}$ = 6 Hz], 5,20 (m, 1H), 5,07 [m, 1H; $^3J_{(OCH=CH)}$ = 6 Hz], 4,05 (s, 2H), 3,86 (s, 3H), 3,72 (s, 3H), 2,0-1,55 (m, 8H), 1,58 (d, d, 3H)].

Analog kann man herstellen:

(E)-2-Propen-1-yloxybenzolsulfonsäure-N-[4-(5-cyclopentyloxycarbonylamino-1-methyl-indol-3-ylmethyl)-3-methoxy-benzoyl]amid [Smp.: 202-205° (aus Dichlormethan/Petrolether), $^1$H-NMR (400 MHz, $CDCl_3$): 8,96 (s, 1H), 8,20 (d, d, 1H), 7,59 (m, 2H), 7,35 (d, 1H), 7,26-7,13 (m, 5H), 7,08 (d, 1H), 6,80 (s, 1H), 6,49 (s, 1H), 6,34 [d, q, 1H; $^3J_{(OCH=CH)}$ = 12 Hz], 5,45 [d, q, 1H; $^3J_{(OCH=CH)}$ = 12 Hz], 5,21 (m, 1H), 4,07 (s, 2H), 3,88 (s, 3H), 3,74 (s, 3H), 1,95-1,58 (m, 8H), 1,60 (d, d, 3H)].

Die Ausgangsmaterialien können z. B. wie folgt hergestellt werden:

a) Eine Lösung von 1,18 g N-(3-Dimethylaminopropyl)-N'-ethyl-carbodiimidhydrochlorid, 1,26 g (Z)-2-Propen-1-yloxybenzolsulfonsäureamid und 0,74 g 4-Dimethylaminopyridin in 60 ml Dichlormethan wird unter Rühren und Einleiten von Argon bei Raumtemperatur mit einer heissen Lösung von 2,0 g 3-Methoxy-4-(1-methyl-5-nitro-indol-3-ylmethyl)-benzoesäure in 100 ml Tetrahydrofuran versetzt. Die gelbe Lösung wird 12 Stunden bei Raumtemperatur gerührt, wobei schon nach 5 Minuten Kristallisation einsetzt. Die gelben Kristalle werden abfiltriert und mit 10 ml Dichlormethan gewaschen. Das (Z)-2-Propen-1-yloxybenzolsulfonsäure-N-[4-(1-methyl-5-nitro-indol-3-ylmethyl)-3-methoxy-benzoyl]amid

schmilzt bei 180-185°.

Analog kann hergestellt werden:

(E)-2-Propen-1-yloxybenzolsulfonsäure-N-[4-(1-methyl-5-nitro-indol-3-ylmethyl)-3-methoxy-benzoyl]amid- [Smp.: 210-215° (aus Tetrahydrofuran/Dichlormethan)].

b) Eine Lösung von 1,0 g (Z)-2-Propen-1-yloxybenzolsulfonsäure-N-[4-(1-methyl-5-nitro-indol-3-ylmethyl)-3-methoxy-benzoyl]amid in 50 ml Dimethylsulfoxid wird nach Zusatz von 1,0 g Lindlar-Katalysator und 5 mg 1,8-Dihydroxy-3,6-dithia-oktan bis zum Stillstand (7 Stunden) bei 20-22° unter Normaldruck hydriert. Zur Abtrennung des Katalysators wird das Gemisch durch Hyflo Super Cel filtriert. Das Filtrat wird unter 0,01 Torr bei 30° eingedampft. Den Rückstand chromatographiert man an 80g Silicagel. Die Fraktionen 1-5, eluiert mit je 60 ml Ethylacetat, werden verworfen. Die Fraktionen 6-13, eluiert mit dem gleichen Lösungsmittel, werden vereinigt und unter 11 Torr bei 40° eingedampft. Den Rückstand verrührt man mit Diethylether, wobei das (Z)-2-Propen-1-yloxybenzolsulfonsäure-N-[4-(5-amino-1-methyl-indol-3-ylmethyl)-3-methoxybenzoyl]amid auskristallisiert [gelbliche Kristalle, Smp.: 135-140°, $^1$H-NMR (400 MHz, DMSO-$d_6$): 7,88 (d, d, 1H), 7,54 (d, 1H), 7,44 (t, d, 1H), 7,37 (d, d, 1H), 7,10 (t, d, 1H), 7,06 (d, 1H), 7,02 (d, d, 1H), 6,99 (d, 1H), 6,81 (s, 1H), 6,63 (d, 1H), 6,56 (d, d, 1H), 6,47 [m, 1H; $^3J_{(OCH=CH)}$ = 6Hz], 4,88 [m, 1H; $^3J_{(OCH-CH)}$ = 6 Hz], 3,86 (s, 2H), 3,84 (s, 3H), 3,60 (s, 3H), 1,56 (d, d, 3H)].

Analog kann hergestellt werden:

(E)-2-Propen-1-yloxybenzolsulfonsäure-N-[4-(5-amino-1-methyl-indol-3-ylmethyl)-3-methoxy-benzoyl]-amid [Smp.: 140-145° (nach Verreiben mit Diethylether), $^1$H-NMR (400 MHz, DMSO-$d_6$, 60°): 7,90 (d, d, 1H), 7,52 (m, 2H), 7,37 (d, d, 1H), 7,16 (t, d, 1H), 7,12 (d, d, 1H), 7,08 (d, 1H), 7,03 (d, 1H), 6,85 (s, 1H), 6,66 (d, 1H), 6,58 (d, d, 1H), 6,54 [q, d, 1H; $^3J_{(OCH=CH)}$ = 12 Hz], 5,27 [q, d, 1H; $^3J_{(OCH=CH)}$ = 12 Hz], 3,87 (s, 5H), 3,61 (s, 3H), 1,52 (d, d, 3H)].

c) Eine Lösung von 21,3 g 2-Allyloxybenzolsulfonsäureamid in 100 ml Dimethylsulfoxid wird unter Rühren innert 5 Minuten mit 14,6 g Kalium-tert.-butanolat versetzt, wobei durch Kühlung die Temperatur unter 30° gehalten wird. Die Mischung wird 15 Stunden bei Raumtemperatur gerührt. Die entstandene Suspension wird auf 300 ml Wasser gegossen und das Gemisch mit 2 N-Salzsäure auf pH 7 gestellt. Die Suspension wird 15 Minuten bei 10-15° gerührt und filtriert. Die weissen Kristalle (Filterkuchen) werden mit 50 ml Wasser gewaschen und 15 Stunden unter 0,1 Torr bei 40° getrocknet. Das (Z)-2-Propen-1-yloxybenzolsulfonsäureamid schmilzt bei 106-107° [$^1$H-NMR (400 MHz, $CDCl_3$): $^3J_{Olefin}$ = 5,9 Hz].

d) Eine Lösung von 2,5 g 2-(2-Chlorpropyloxy)benzolsulfonsäureamid in 27 ml tert.-Butanol wird unter Rühren mit 4,2 g Kalium-tert.-butanolat versetzt. Man rührt die Mischung bei 80° Innentemperatur, kühlt sie ab und versetzt die weisse Suspension mit einer Mischung aus 25 ml Eiswasser und 5 ml konzentrierter Salzsäure. Man extrahiert das Gemisch dreimal mit je 70 ml Ethylacetat, wäscht die vereinigten organischen Phasen nacheinander mit 40 ml 2 N-Natriumhydrogencarbonatlösung und dreimal mit je 40 ml gesättigter Natriumchloridlösung, trocknet sie über Magnesiumsulfat und engt sie unter 11 Torr bei 40° zur Trockne ein. Man verrührt den kristallinen Rückstand mit 50 ml Diethylether und filtriert die Kristalle ab. Das (E)-2-Propen-1-yloxybenzolsulfonsäureamid schmilzt bei 145-146° [$^1$H-NMR (400 MHz, $CDCl_3$): $^3J_{Olefin}$ = 12,4 Hz].

e) Eine Mischung aus 10,38 g 2-Hydroxybenzolsulfonsäureamid, 16,6 g Kaliumcarbonat und 1,0 g Kaliumiodid in 45 ml wasserfreiem N,N-Dimethylformamid wird unter Rühren und Kühlung mit einer Lösung von 8,64 ml Bromaceton (70 %) in 10 ml wasserfreiem N,N-Dimethylformamid versetzt. Die Suspension wird eine Stunde bei Raumtemperatur gerührt und dann auf ein Gemisch von 100 ml Eiswasser und 15 ml konzentrierter Salzsäure gegossen. Die Mischung wird viermal mit je 50 ml Ethylacetat extrahiert. Die vereinigten organischen Extrakte werden viermal mit je 25 ml gesättigter Natriumchloridlösung gewaschen, über Magnesiumsulfat getrocknet und unter 11 Torr bei 40° eingeengt, wobei das 2-(2-Oxopropyloxy)benzolsulfonsäureamid auskristallisiert (Smp.: 158-159°).

f) Zu einer Suspension von 11,5 g 2-(2-Oxopropyloxy)benzolsulfonsäureamid in 50 ml Ethanol und 6 ml Wasser setzt man unter Rühren und Kühlung portionsweise 1,0 g Natriumborhydrid zu. Die Suspension wird 4 Stunden bei Raumtemperatur gerührt, mit 5 ml Aceton versetzt und unter 11 Torr bei 50° zur Trockne eingeengt. Den Rückstand versetzt man mit 150 ml Ethylacetat und 50 ml Wasser. Durch Zusatz von 2 N-Salzsäure wird der pH des Gemisches auf 2-3 eingestellt. Man trennt die organische Phase ab, wäscht sie nacheinander zweimal mit je 50 ml Wasser und zweimal mit je 50 ml gesättigter Natriumchloridlösung, trocknet sie über Natriumsulfat und engt sie unter 11 Torr zur Trockne ein. Den Rückstand kristallisiert man aus Aceton/Diethylether. Das 2-(2-Hydroxypropyloxy)benzolsulfonsäureamid schmilzt bei 110-111°.

g) Eine Suspension von 11,6 g 2-(2-Hydroxypropyloxy)benzolsulfonsäureamid in 300 ml Trichlormethan wird unter Rühren bei 25° mit 9,0 ml 1-Chlor-1-(N,N-dimethylamino)-2-methyl-propen versetzt. Die Mischung wird 30 Minuten bei 25° gerührt, mit 200 ml Trichlormethan verdünnt und auf 1000 ml

Eiswasser gegossen. Die organische Phase wird abgetrennt, zweimal mit je 100 ml Wasser gewaschen, über Natriumsulfat getrocknet und unter 11 Torr bei 40° eingeengt. Der Rückstand wird an 600 g Silicagel mit Toluol/-Ethylacetat (4:1) flash-chromatographiert. Das Eluat wird unter 11 Torr bei 50° eingeengt. Man verreibt den Rückstand mit Diethylether und filtriert die Kristalle ab. Das 2-(2-Chlorpropyloxy)benzolsulfonsäureamid schmilzt bei 113-114°.

Beispiele A bis H: Pharmazeutische Präparate.

Unter dem Ausdruck "Wirkstoff" ist nachstehend eine Verbindung I, in freier Form oder in Form eines pharmazeutisch verwendbaren Salzes, zu verstehen, insbesondere eine solche Verbindung, die als Produkt in den Beispielen 1 bis 3 beschrieben ist, z.B. das 2-Vinyloxybenzolsulfonsäure-N-[4-(5-cyclopentyloxycarbonylamino-1-methyl-indol-3-ylmethyl)-3-methoxy-benzoyl]amid.

Beispiel A: Eine treibmittelhaltige, feststoffaerosolbildende Inhalationssuspension, enthaltend 0,1 Gewichtsprozent Wirkstoff.

| Zusammensetzung | |
|---|---|
| | Gewichtsprozent |
| Wirkstoff, mikronisiert | 0,1 |
| Sorbitantrioleat | 0,5 |
| Treibmittel A (Trichlortrifluorethan) | 4,4 |
| Treibmittel B (Dichlordifluormethan und | 15,0 |
| 1,2-Dichlortetrafluorethan) | 80,0 |

Der Wirkstoff wird unter Feuchtigkeitsausschluss mit Hilfe eines üblichen Homogenisators unter Zusatz des Sorbitantrioleats im Trichlortrifluorethan suspendiert und die Suspension in einen mit einem Dosierventil versehenen Aerosolbehälter abgefüllt. Der Behälter wird verschlossen und unter Druck mit dem Treibmittel B aufgefüllt.

Beispiel B: Eine zur Inhalation geeignete, etwa zweiprozentige, wässrige Lösung des Wirkstoffs in Form seines Natrium- oder Kalium-salzes.

| Zusammensetzung | |
|---|---|
| Wirkstoff (K- oder Na-Salz) | 2000 mg |
| Ethylendiamintetraessigsäure-Dinatriumsalz | 10 mg |
| Benzalkoniumchlorid | 10 mg |
| Wasser, frisch destilliert | ad 100 ml |
| Treibmittel | nach Bedarf |

Der Wirkstoff wird in etwa 60 ml frisch destilliertem Wasser gelöst und der Stabilisator (Ethylendiamintetraessigsäure-Dinatriumsalz) und das Konservierungsmittel (Benzalkoniumchlorid) werden hinzugegeben. Nach vollständiger Auflösung aller Komponenten wird die erhaltene Lösung auf 100 ml aufgefüllt und in Druckfläschchen abgefüllt. Die Fläschchen werden gasdicht verschlossen. Das Treibmittel wird nach Bedarf gasförmig unter Druck oder in flüssiger Form zugegeben.

Beispiel C: Eine Salbe, enthaltend 0,05 Gewichtsprozent Wirkstoff.

| Zusammensetzung | |
|---|---|
| | Gewichtsprozent |
| Wirkstoff | 0,05 |
| Vaseline | 45,00 |
| Paraffinöl | 19,60 |
| Cetylalkohol | 5,00 |
| Bienenwachs | 5,00 |
| Sorbitan-sesquioleat | 5,00 |
| p-Hydroxybenzoesäureester | 0,20 |
| Wasser, entmineralisiert | 20,15 |

Die Fettstoffe und Emulgatoren werden zusammengeschmolzen. Das Konservierungsmittel wird in Wasser gelöst und die Lösung in die Fettschmelze bei erhöhter Temperatur einemulgiert. Nach dem Erkalten wird eine Suspension des Wirkstoffs in einem Teil der Fettschmelze in die Emulsion eingearbeitet.

Beispiel D: Augentropfen, enthaltend 0,3 Gewichtsprozent Wirkstoff.

| Zusammensetzung (10000 Flaschen à 10 ml) | |
|---|---|
| | Gewichtsprozent |
| Wirkstoff | 0,30 |
| Dinatriumphosphat | 0,31 |
| Zitronensäure | 0,15 |
| Natriumchlorid | 0,35 |
| Natriumpyrosulfit | 0,10 |
| Benzalkoniumchlorid | 0,01 |
| Wasser, entmineralisiert | 98,78 |

Der Wirkstoff und alle angegebenen Zusatzstoffe werden unter einer Stickstoffatmosphäre in 80 l entmineralisiertes Wasser eingerührt. Nach der vollständigen Auflösung aller Bestandteile wird die Lösung mit entmineralisiertem Wasser auf 100 l aufgefüllt, in einem Autoklaven bei 120° während 20 Minuten sterilisiert und anschliessend unter sterilen Bedingungen durch ein Membranfilter (Porendurchmesser: 0,2 μm) filtriert. Je 10 ml des Filtrats werden unter aseptischen Bedingungen in eine Flasche mit Tropfpipettenverschluss abgefüllt.

Beispiel E: Tabletten, enthaltend je 50 mg Wirkstoff.

| Zusammensetzung (10000 Tabletten) | |
|---|---|
| Wirkstoff | 500,0 g |
| Lactose | 500,0 g |
| Kartoffelstärke | 352,0 g |
| Gelatine | 8,0 g |
| Talk | 60,0 g |
| Magnesiumstearat | 10,0 g |
| Siliciumdioxid (hochdispers) | 20,0 g |
| Ethanol | q.s. |

Der Wirkstoff wird mit der Lactose und 292 g der Kartoffelstärke vermischt und die Mischung mit einer ethanolischen Lösung der Gelatine befeuchtet und durch ein Sieb granuliert. Nach dem Trocknen mischt man den Rest der Kartoffelstärke, das Magnesiumstearat, den Talk und das Siliciumdioxid zu und presst das Gemisch zu Tabletten von je 145 mg Gewicht und 50 mg Wirkstoffgehalt, die gewünschtenfalls mit Teilkerben zur feineren Anpassung der Dosierung versehen sein können.

Beispiel F: Lacktabletten, enthaltend je 100 mg Wirkstoff.

| Zusammensetzung (1000 Lacktabletten) | |
|---|---|
| Wirkstoff | 100,0 g |
| Lactose | 100,0 g |
| Maisstärke | 70,0 g |
| Talk | 8,5 g |
| Calciumstearat | 1,5 g |
| Hydroxypropylmethylcellulose | 2,36 g |
| Schellack | 0,64 g |
| Wasser | q.s. |
| Dichlormethan | q.s. |

Der Wirkstoff, die Lactose und 40 g der Maisstärke werden gemischt. Das Gemisch wird mit einem Kleister, hergestellt aus 15 g Maisstärke und Wasser (unter Erwärmen), befeuchtet und granuliert. Das Granulat wird getrocknet und der Rest der Maisstärke, der Talk und das Calciumstearat werden mit dem Granulat vermischt. Das Gemisch wird zu Tabletten (Gewicht: je 280 mg) verpresst und diese werden mit einer Lösung der Hydroxypropylmethylcellulose und des Schellacks in Dichlormethan lackiert (Endgewicht der Lacktabletten: je 283 mg).

Beispiel G: Gelatinesteckkapseln, enthaltend je 100 mg Wirkstoff.

| Zusammensetzung (1000 Kapseln) | |
|---|---|
| Wirkstoff | 100,0 g |
| Lactose | 250,0 g |
| mikrokristalline Cellulose | 30,0 g |
| Natriumlaurylsulfat | 2,0 g |
| Magnesiumstearat | 8,0 g |

Das Natriumlaurylsulfat wird durch ein Sieb mit einer Maschenweite von 0,2 mm zu dem lyophilisierten Wirkstoff hinzugesiebt. Beide Komponenten werden innig gemischt. Dann wird zunächst die Lactose durch ein Sieb mit einer Maschenweite von 0,6 mm und anschliessend die mikrokristalline Cellulose durch ein Sieb mit einer Maschenweite von 0,9 mm hinzugesiebt. Dann werden alle vier Komponenten 10 Minuten innig gemischt. Zuletzt wird das Magnesiumstearat durch ein Sieb mit einer Maschenweite von 0,8 mm hinzugesiebt. Nach weiterem Mischen (3 Minuten) werden je 390 mg der erhaltenen Formulierung in Gelatinesteckkapseln der Grösse 0 abgefüllt.

Beispiel H: Eine Injektions- oder Infusionslösung, enthaltend 5 mg Wirkstoff je 2,5 ml-Ampulle.

| Zusammensetzung (1000 Ampullen) | |
|---|---|
| Wirkstoff | 5,0 g |
| Natriumchlorid | 22,5 g |
| Phosphatpufferlösung (pH:7,4) | 300,0 g |
| entmineralisiertes Wasser | ad 2500,0 ml |

Der Wirkstoff und das Natriumchlorid werden in 1000 ml entmineralisiertem Wasser gelöst. Die Lösung wird durch ein Mikrofilter filtriert. Man versetzt das Filtrat mit der Phosphatpufferlösung und füllt das Gemisch mit entmineralisiertem Wasser auf 2500 ml auf. Zur Herstellung von Dosiseinheitsformen werden je 2,5 ml des Gemisches in Glasampullen abgefüllt, die dann je 5 mg Wirkstoff enthalten.

**Patentansprüche**

**Patentansprüche für folgende Vertragsstaaten : AT, BE, CH, DE, DK, FR, GB, IT, LI, LU, NL, SE**

**1.** Eine Verbindung der Formel

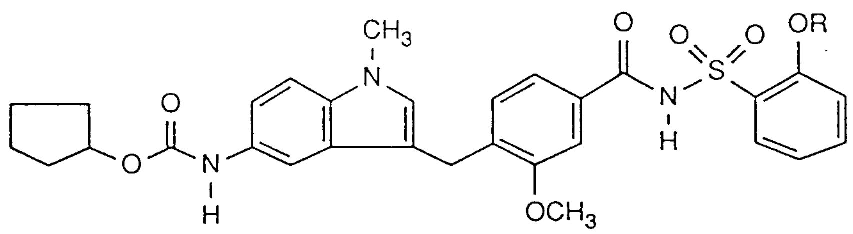

(I),

worin R geradkettiges $C_2$-$C_4$-Alk-1-en-1-yl bedeutet, in freier Form oder in Salzform.

**2.** Eine Verbindung gemäss Anspruch 1 der Formel I, worin R Vinyl, (Z)-Propen-1-yl oder (E)-Propen-1-yl bedeutet, in freier Form oder in Salzform.

**3.** Eine Verbindung gemäss Anspruch 1 der Formel I, worin R Vinyl oder (Z)-Propen-1-yl bedeutet, in freier Form oder in Salzform.

**4.** 2-Vinyloxybenzolsulfonsäure-N-[4-(5-cyclopentyloxycarbonylamino-1-methyl-indol-3-ylmethyl)-3-methoxy-benzoyl]amid, in freier Form oder in Salzform.

**5.** (Z)-2-Propen-1-yloxybenzolsulfonsäure-N-[4-(5-cyclopentyloxycarbonylamino-1-methyl-indol-3-ylmethyl)-3-methoxy-benzoyl]amid, in freier Form oder in Salzform.

**6.** (E)-2-Propen-1-yloxybenzolsulfonsäure-N-[4-(5-cyclopentyloxycarbonylamino-1-methyl-indol-3-ylmethyl)-3-methoxy-benzoyl]amid, in freier Form oder in Salzform.

**7.** Eine Verbindung gemäss einem der Ansprüche 1 bis 6, in freier Form oder in pharmazeutisch verwendbarer Salzform, zur Anwendung in einem Verfahren zur therapeutischen Behandlung des menschlichen oder tierischen Körpers.

**8.** Eine Verbindung gemäss einem der Ansprüche 1 bis 6, in freier Form oder in pharmazeutisch verwendbarer Salzfonm, zur Anwendung als Antiallergikum.

**9.** Ein pharmazeutisches Präparat, als Wirkstoff enthaltend eine Verbindung gemäss einem der Ansprüche 1 bis 6, in freier Form oder in pharmazeutisch verwendbarer Salzform, gegebenenfalls neben üblichen pharmazeutischen Hilfsstoffen.

**10.** Ein pharmazeutisches Präparat gemäss Anspruch 9, dadurch gekennzeichnet, dass es antiallergisch wirksam ist.

**11.** Verfahren zur Herstellung einer Verbindung der Formel

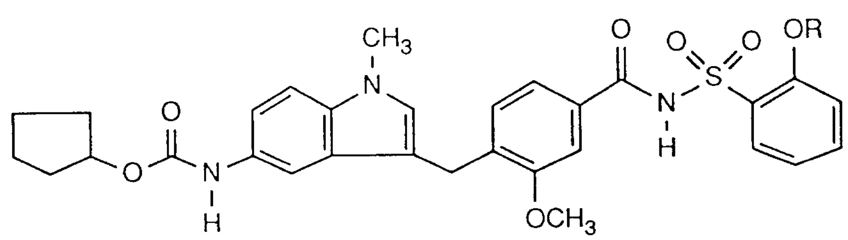

(I),

worin R geradkettiges $C_2$-$C_4$-Alk-1-en-1-yl bedeutet, in freier Form oder in Salzform, dadurch gekennzeichnet, dass man

a) eine Verbindung der Formel

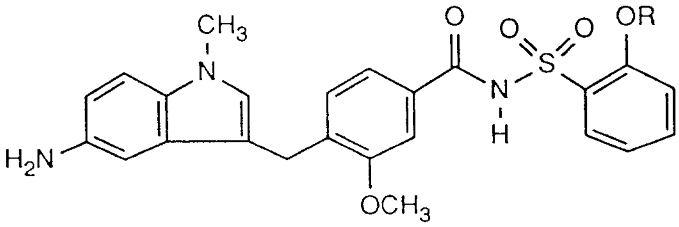

(II),

oder ein Salz davon, mit einer Verbindung der Formel

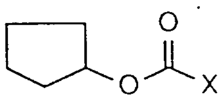

(III),

worin X eine nucleofuge Abgangsgruppe ist, unter Abspaltung einer Verbindung H-X umsetzt, oder
b) in einer Verbindung der Formel

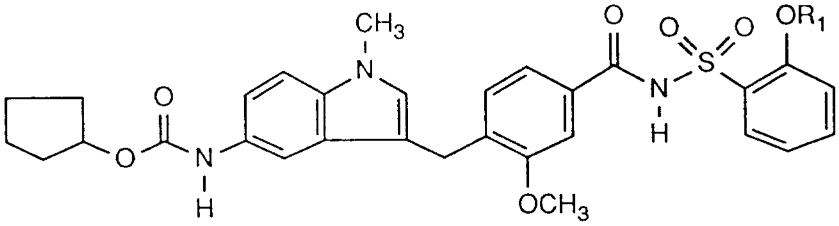

(IV),

worin $R_1$ eine in R überführbare Gruppe bedeutet, oder einem Salz davon, $R_1$ in R überführt, und gewünschtenfalls jeweils ein verfahrensgemäss erhältliches Isomerengemisch in die Komponenten auftrennt und/oder eine verfahrensgemäss erhältliche freie Verbindung I in ein Salz überführt oder ein verfahrensgemäss erhältliches Salz einer Verbindung I in die freie Verbindung I oder in ein anderes Salz umwandelt.

**12.** Verfahren zur Herstellung eines pharmazeutischen Präparats gemäss Anspruch 9 oder 10, dadurch gekennzeichnet, dass man den Wirkstoff, gegebenenfalls unter Beimischung von üblichen pharmazeutischen Hilfsstoffen, zu einem pharmazeutischen Präparat verarbeitet.

**13.** Verfahren gemäss Anspruch 12 zur Herstellung eines antiallergisch wirksamen pharmazeutischen Präparats gemäss Anspruch 10, dadurch gekennzeichnet, dass man einen antiallergisch wirksamen Wirkstoff wählt.

**14.** Verwendung einer Verbindung gemäss einem der Ansprüche 1 bis 6, in freier Form oder in pharmazeutisch verwendbarer Salzform, zur Herstellung eines pharmazeutischen Präparats.

**15.** Verwendung einer Verbindung gemäss einem der Ansprüche 1 bis 6, in freier Form oder in pharmazeutisch verwendbarer Salzform, zur Herstellung eines pharmazeutischen Präparats auf nicht-chemischen Wege.

**16.** Verwendung einer Verbindung gemäss einem der Ansprüche 1 bis 6 zur Herstellung eines Antiallergikums.

**Patentansprüche für folgende Vertragsstaaten : ES, GR**

1. Verfahren zur Herstellung einer Verbindung der Formel

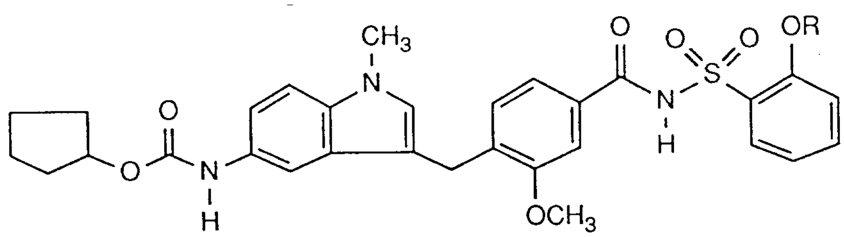

(I),

worin R geradkettiges $C_2$-$C_4$-Alk-1-en-1-yl bedeutet, in freier Form oder in Salzform, dadurch gekennzeichnet, dass man

a) eine Verbindung der Formel

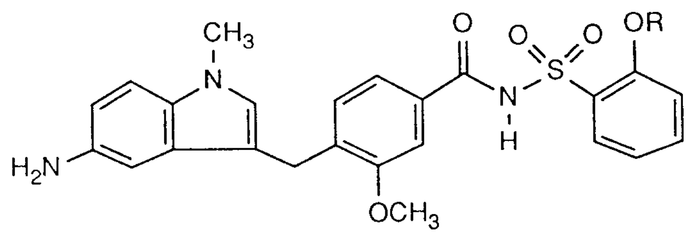

(II),

oder ein Salz davon, mit einer Verbindung der Formel

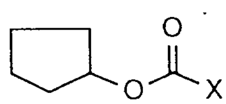

(III),

worin X eine nucleofuge Abgangsgruppe ist, unter Abspaltung einer Verbindung H-X umsetzt, oder

b) in einer Verbindung der Formel

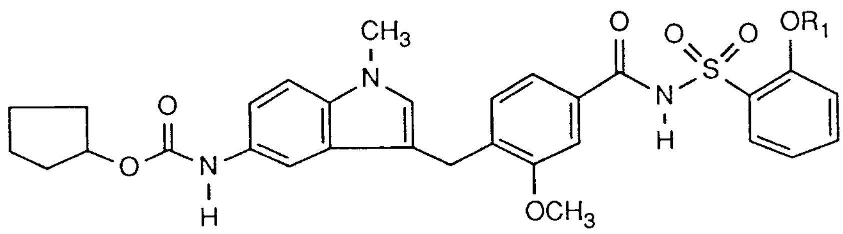

(IV),

worin $R_1$ eine in R überführbare Gruppe bedeutet, oder einem Salz davon, $R_1$ in R überführt, und gewünschtenfalls jeweils ein verfahrensgemäss erhältliches Isomerengemisch in die Komponenten auftrennt und/oder eine verfahrensgemäss erhältliche freie Verbindung I in ein Salz überführt oder ein verfahrensgemäss erhältliches Salz einer Verbindung I in die freie Verbindung I oder in ein anderes Salz umwandelt.

2. Verfahren gemäss Anspruch 1, dadurch gekennzeichnet, dass man eine Verbindung der Formel I, worin R Vinyl, (Z)-Propen-1-yl oder (E)-Propen-1-yl bedeutet, in freier Form oder in Salzform, herstellt.

3. Verfahren gemäss Anspruch 1, dadurch gekennzeichnet, dass man eine Verbindung der Formel I, worin R Vinyl oder (Z)-Propen-1-yl bedeutet, in freier Form oder in Salzform, herstellt.

4. Verfahren gemäss Anspruch 1, dadurch gekennzeichnet, dass man 2-Vinyloxvbenzolsulionsäure-N-[4-(5-cyclopentyloxycarbonylamino-1-methyl-indol-3-ylmethyl)-3-methoxy-benzoyl]amid, in freier Form oder in Salzform, herstellt.

5. Verfahren gemäss Anspruch 1, dadurch gekennzeichnet, dass man (Z)-2-Propen-1-yloxybenzolsulfonsäure-N-[4-(5-cyclopentyloxycarbonylamino-1-methyl-indol-3-ylmethyl)-3-methoxy-benzoyl]amid, in freier Form oder in Salzform, herstellt.

6. Verfahren gemäss Anspruch 1, dadurch gekennzeichnet, dass man (E)-2-Propen-1-yloxybenzolsulfonsäure-N-[4-(5-cyclopentyloxycarbonylamino-1-methyl-indol-3-ylmethyl)-3-methoxy-benzoyl]amid, in freier Form oder in Salzform, herstellt.

7. Verfahren zur Herstellung eines pharmazeutischen Präparats, dadurch gekennzeichnet, dass man
a) eine Verbindung der Formel

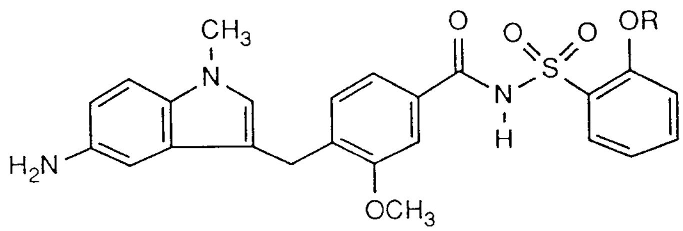

(II),

oder ein Salz davon, mit einer Verbindung der Formel

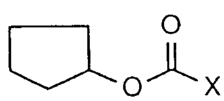

(III),

worin X eine nucleofuge Abgangsgruppe ist, unter Abspaltung einer Verbindung H-X umsetzt, oder
b) in einer Verbindung der Formel

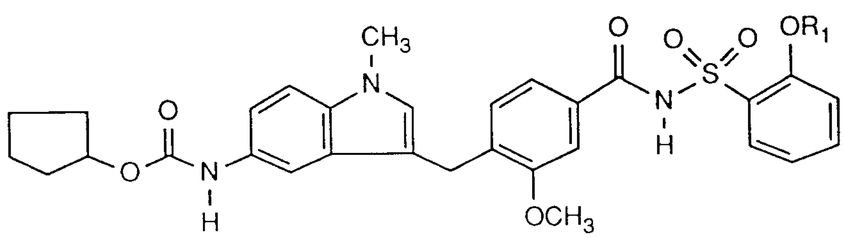

(IV),

worin $R_1$ eine in R überführbare Gruppe bedeutet, oder einem Salz, davon $R_1$ in R überführt, und gewünschtenfalls jeweils ein verfahrensgemäss erhältliches Isomerengemisch in die Komponenten auftrennt und/oder eine verfahrensgemäss erhältliche freie Verbindung I in ein pharmazeutisch verwendbares Salz überführt oder ein verfahrensgemäss erhältliches pharmazeutisch verwendbares Salz einer Verbindung I in die freie Verbindung I oder in ein anderes pharmazeutisch verwendbares Salz umwandelt, und eine auf diese Weise erhaltene Verbindung der Formel

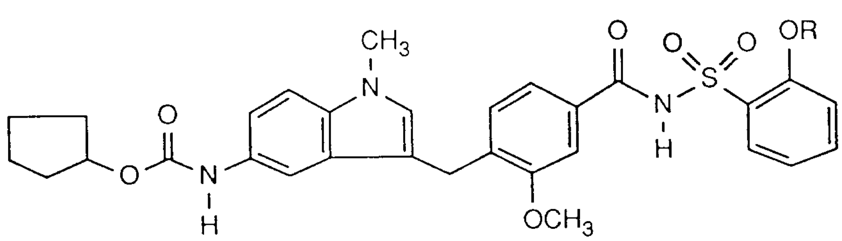

(I),

worin R geradkettiges $C_2$-$C_4$-Alk-1-en-1-yl bedeutet, in freier Form oder in pharmazeutisch verwendbarer Salzform, gegebenenfalls unter Beimischung von üblichen pharmazeutischen Hilfsstoffen, zu einem pharmazeutischen Präparat verarbeitet.

**8.** Verfahren zur Herstellung eines pharmazeutischen Präparats, dadurch gekennzeichnet, dass man eine Verbindung, erhältlich gemäss einem der Ansprüche 1 bis 6, in freier Form oder in Form eines pharmazeutisch venwendbaren Salzes, gegebenenfalls unter Beimischung von üblichen pharmazeutischen Hilfsstoffen, zu einem pharmazeutischen Präparat verarbeitet.

**9.** Verfahren gemäss Anspruch 7 oder 8 zur Herstellung eines antiallergisch wirksamen pharmazeutischen Präparats, dadurch gekennzeichnet, dass man einen antiallergisch wirksamen Wirkstoff wählt.

**10.** Verwendung einer Verbindung, erhältlich gemäss einem der Ansprüche 1 bis 6, in freier Form oder in Form eines pharmazeutisch verwendbaren Salzes, zur Herstellung eines pharmazeutischen Präparats.

**11.** Verwendung einer Verbindung, erhältlich gemäss einem der Ansprüche 1 bis 6, in freier Form oder in Form eines pharmazeutisch verwendbaren Salzes, zur Herstellung eines pharmazeutischen Präparats auf nicht-chemischem Wege.

**12.** Verwendung einer Verbindung, erhältlich gemäss einem der Ansprüche 1 bis 6, zur Herstellung eines Antiallergikums.

**Claims**

**Claims for the following Contracting States : AT, BE, CH, DE, DK, FR, GB, IT, LI, LU, NL, SE**

**1.** A compound of formula

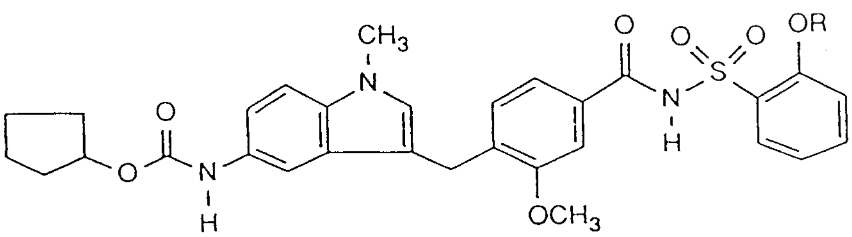 (I),

wherein R is straight-chain $C_2$-$C_4$alk-1-en-1-yl, in free form or in the form of a salt.

**2.** A compound according to claim 1 of formula I, in which R is vinyl, (Z)-propen-1-yl or (E)-propen-1-yl, in free form or in the form of a salt.

**3.** A compound according to claim 1 of formula I, in which R is vinyl or (Z)-propen-1-yl, in free form or in the form of a salt.

**4.** 2-Vinyloxybenzenesulfonic acid N-[4-(5-cyclopentyloxycarbonylamino-1-methyl-indol-3-ylmethyl)-3-methoxybenzoyl]amide, in free form or in the form of a salt.

**5.** (Z)-2-Propen-1-yloxybenzenesulfonic acid N-[4-(5-cyclopentyloxycarbonylamino-1-methyl-indol-3-ylmethyl)-3-methoxy-benzoyl]amide, in free form or in the form of a salt.

**6.** (E)-2-Propen-1-yloxybenzenesulfonic acid N-[4-(5-cyclopentyloxycarbonylamino-1-methyl-indol-3-ylmethyl)-3-methoxy-benzoyl]amide, in free form or in the form of a salt.

**7.** A compound according to any one of claims 1 to 6, in free form or in the form of a pharmaceutically acceptable salt, for use in a method for the therapeutic treatment of the human or animal body.

**8.** A compound according to any one of claims 1 to 6, in free form or in the form of a pharmaceutically acceptable salt, for use as an antiallergic.

**9.** A pharmaceutical composition comprising as active ingredient a compound according to any one of claims 1 to 6, in free form or in the form of a pharmaceutically acceptable salt, where appropriate together with customary pharmaceutical adjuncts.

**10.** A pharmaceutical composition according to claim 9 that is antiallergically effective.

**11.** A process for the manufacture of a compound of formula

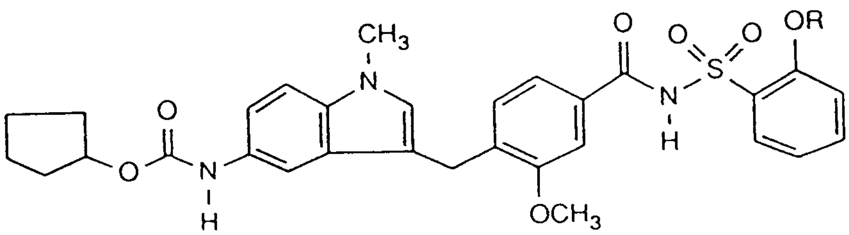

(I),

wherein R is straight-chain $C_2$-$C_4$alk-1-en-1-yl, in free form or in the form of a salt, which process comprises

a) reacting a compound of formula

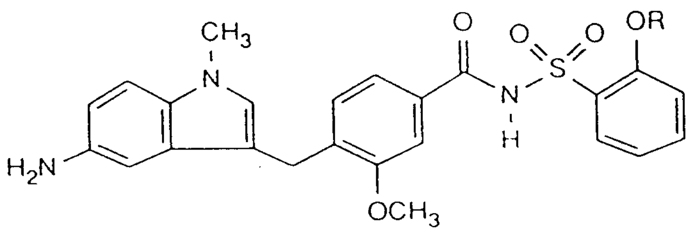

(II),

or a salt thereof, with a compound of formula

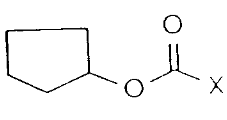

(III),

wherein X is a nucleofugal leaving group, with removal of a compound H-X, or

b) in a compound of formula

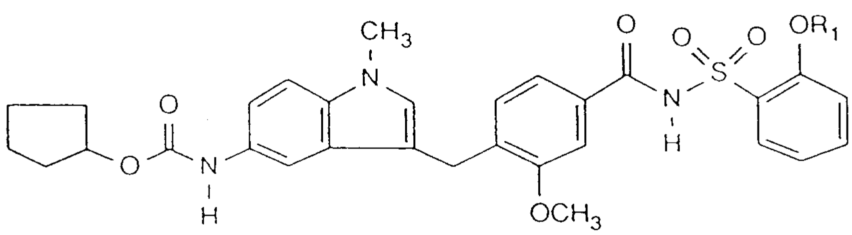

(IV),

wherein $R_1$ is a group that can be converted into R, or in a salt thereof, converting $R_1$ into R, and, if desired, in each case separating a mixture of isomers obtainable in accordance with the process into its components and/or converting a free compound I obtainable in accordance with the process into a salt or converting a salt of a compound I obtainable in accordance with the process into the free compound I or into a different salt.

**12.** A process for the manufacture of a pharmaceutical composition according to claim 9 or 10, which process comprises processing the active ingredient, where appropriate with the admixture of customary pharmaceutical adjuncts, into a pharmaceutical composition.

**13.** A process according to claim 12 for the manufacture of an antiallergically effective pharmaceutical composition according to claim 10, wherein an antiallergically effective active ingredient is selected.

**14.** The use of a compound according to any one of claims 1 to 6, in free form or in the form of a pharmaceutically acceptable salt, for the manufacture of a pharmaceutical composition.

**15.** The use of a compound according to any one of claims 1 to 6, in free form or in the form of a pharmaceutically acceptable salt, for the manufacture of a pharmaceutical composition by non-chemical methods.

**16.** The use of a compound according to any one of claims 1 to 6 for the manufacture of an antiallergic.

**Claims for the following Contracting States : ES, GR**

**1.** A process for the manufacture of a compound of formula

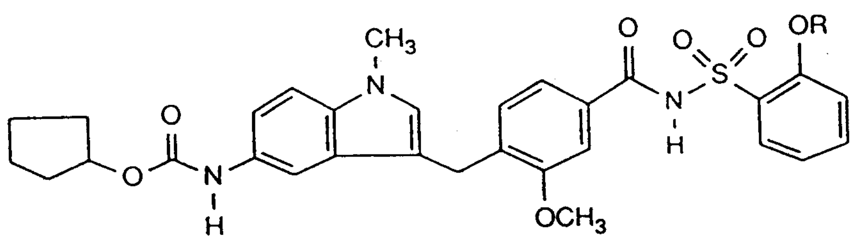

(I),

wherein R is straight-chain $C_2$-$C_4$alk-1-en-1-yl, in free form or in the form of a salt, which process comprises

a) reacting a compound of formula

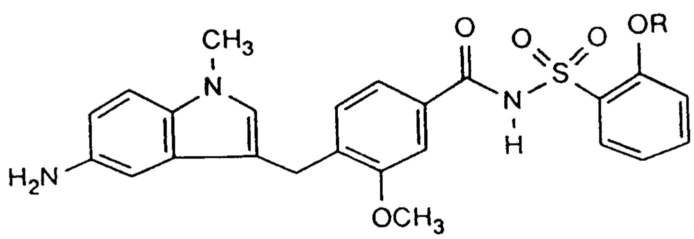

(II),

or a salt thereof, with a compound of formula

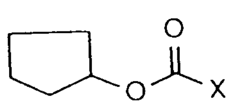

(III),

wherein X is a nucleofugal leaving group, with removal of a compound H-X, or

b) in a compound of formula

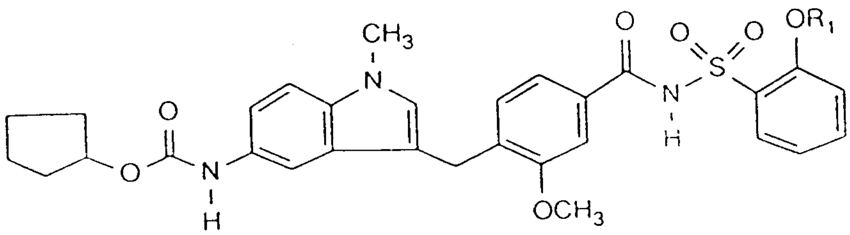

(IV),

wherein $R_1$ is a group that can be converted into R, or in a salt thereof, converting $R_1$ into R, and, if desired, in each case separating a mixture of isomers obtainable in accordance with the process into its components and/or converting a free compound I obtainable in accordance with the process into a salt or converting a salt of a compound I obtainable in accordance with the process into the free compound I or into a different salt.

**2.** A process according to claim 1, wherein there is manufactured a compound of formula I in which R is vinyl, (Z)-propen-1-yl or (E)-propen-1-yl, in free form or in the form of a salt.

**3.** A process according to claim 1, wherein there is manufactured a compound of formula I in which R is vinyl or (Z)-propen-1-yl, in free form or in the form of a salt.

**4.** A process according to claim 1, wherein there is manufactured 2-vinyloxybenzenesulfonic acid N-[4-(5-cyclopentyloxycarbonylamino-1-methyl-indol-3-ylmethyl)-3-methoxy-benzoyl]amide, in free form or in the form of a salt.

**5.** A process according to claim 1, wherein there is manufactured (Z)-2-propen-1-yloxybenzenesulfonic acid N-[4-(5-cyclopentyloxycarbonylamino-1-methyl-indol-3-ylmethyl)-3-methoxy-benzoyl]amide, in free form or in the form of a salt.

**6.** A process according to claim 1, wherein there is manufactured (E)-2-propen-1-yloxybenzenesulfonic acid N-[4-(5-cyclopentyloxycarbonylamino-1-methyl-indol-3-ylmethyl)-3-methoxy-benzoyl]amide, in free form or in the form of a salt.

**7.** A process for the manufacture of a pharmaceutical composition, which process comprises
a) reacting a compound of formula

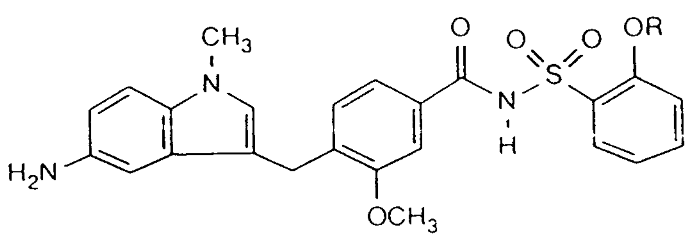

(II),

or a salt thereof, with a compound of formula

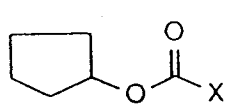

(III),

wherein X is a nucleofugal leaving group, with removal of a compound H-X, or

b) in a compound of formula

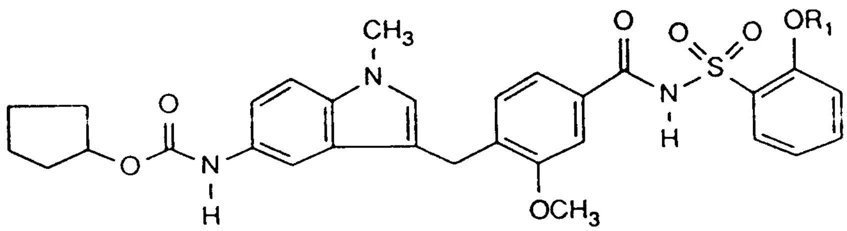

(IV),

wherein $R_1$ is a group that can be converted into R, or in a salt thereof, converting $R_1$ into R, and, if desired, in each case separating a mixture of isomers obtainable in accordance with the process into its components and/or converting a free compound I obtainable in accordance with the process into a pharmaceutically acceptable salt or converting a pharmaceutically acceptable salt of a compound I obtainable in accordance with the process into the free compound I or into a different pharmaceutically acceptable salt, and processing a compound of formula

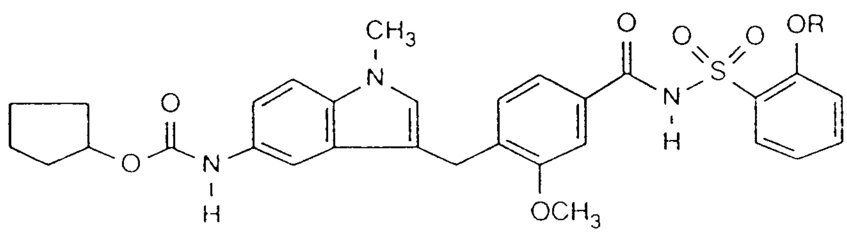

(I)

obtained in that manner, wherein R is straight-chain $C_2$-$C_4$alk-1-en-1-yl, in free form or in the form of a pharmaceutically acceptable salt, into a pharmaceutical composition, where appropriate with the admixture of customary pharmaceutical adjuncts.

**8.** A process for the manufacture of a pharmaceutical composition, which process comprises processing a compound obtainable according to any one of claims 1 to 6, in free form or in the form of a pharmaceutically acceptable salt, into a pharmaceutical composition, where appropriate with the admixture of customary pharmaceutical adjuncts.

**9.** A process according to claim 7 or 8 for the manufacture of an antiallergically effective pharmaceutical composition, wherein an antiallergically effective active ingredient is selected.

**10.** The use of a compound obtainable according to any one of claims 1 to 6, in free form or in the form of a pharmaceutically acceptable salt, for the manufacture of a pharmaceutical composition.

**11.** The use of a compound obtainable according to any one of claims 1 to 6, in free form or in the form of a pharmaceutically acceptable salt, for the manufacture of a pharmaceutical composition by non-chemical methods.

**12.** The use of a compound obtainable according to any one of claims 1 to 6 for the manufacture of an antiallergic.

**Revendications**

**Revendications pour les Etats contractants suivants : AT, BE, CH, DE, DK, FR, GB, IT, LI, LU, NL, SE**

**1.** Composé de formule

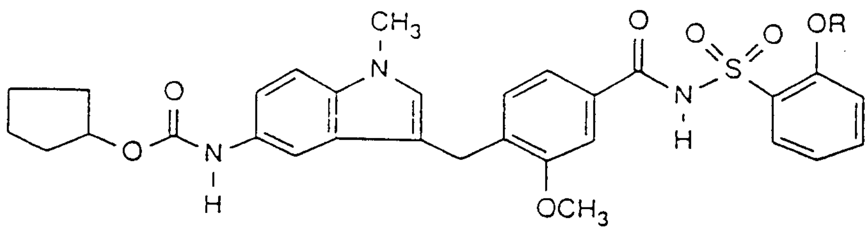

(I),

dans laquelle R représente un $C_2$-$C_4$-alk-1-ène-1-yle à chaîne droite, sous forme libre ou sous forme de sel.

**2.** Composé selon la revendication 1 de formule I, où R représente le vinyle, le (Z)-propène-1-yle ou le (E)-propène-1-yle, sous forme libre ou sous forme de sel.

**3.** Composé selon la revendication 1 de formule I, où R représente le vinyle ou le (Z)-propène-1-yle, sous forme libre ou sous forme de sel.

**4.** N-[4-(5-cyclopentyloxycarbonylamino-1-méthyl-indole-3-ylméthyl)-3-méthoxy-benzoyl]amide de l'acide 2-vinyloxybenzène-sulfonique, sous forme libre ou sous forme de sel.

**5.** N-[4-(5-cyclopentyloxycarbonylamino-1-méthyl-indole-3-ylméthyl)-3-méthoxybenzoyl]amide de l'acide (Z)-2-propène-1-yloxybenzène-sulfonique, sous forme libre ou sous forme de sel.

**6.** N-[4-(5-cyclopentyioxycarbonylamino-1-méthyl-indole-3-ylméthyl)-3-méthoxybenzoyl]amide de l'acide (E)-2-propène-1-yloxybenzène-sulfonique, sous forme libre ou sous forme de sel.

**7.** Composé selon une des revendications 1 à 6, sous forme libre ou sous forme de sel utilisable en pharmacie, pour utilisation dans un procédé pour le traitement thérapeutique des corps humains ou animaux.

**8.** Composé selon une des revendications 1 à 6, sous forme libre ou sous forme de sel utilisable en pharmacie, pour utilisation comme agent antiallergique.

**9.** Préparation pharmaceutique, comportant comme matière active un composé selon une des revendications 1 à 6, sous forme libre ou sous forme de sel utilisable en pharmacie, éventuellement en plus d'adjuvants pharmaceutiques habituels.

**10.** Préparation pharmaceutique selon la revendication 9, caractérisée en ce qu'elle est une matière active antiallergique.

**11.** Procédé pour préparer un composé de formule

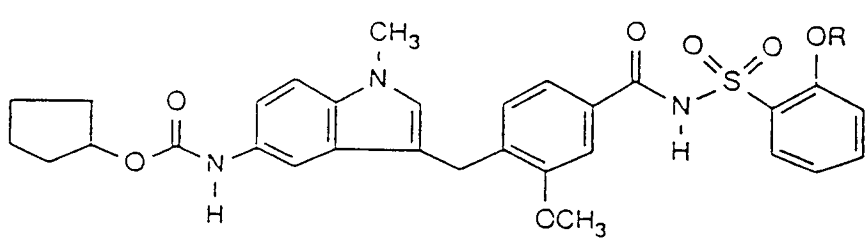

(I),

dans laquelle R représente un $C_2$-$C_4$-alk-1-ène-1-yle à chaîne droite, sous forme libre ou sous forme de sel, caractérisé en ce que

a) on fait réagir un composé de formule

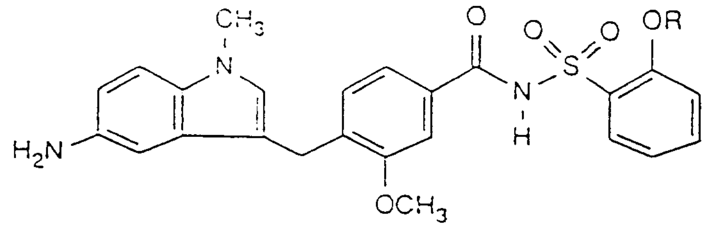

(II),

ou un sel de celui-ci avec un composé de formule

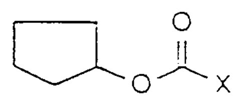

(III),

où X est un groupe éliminable, nucléofuge, avec séparation d'un composé H-X, ou

b) dans un composé de formule

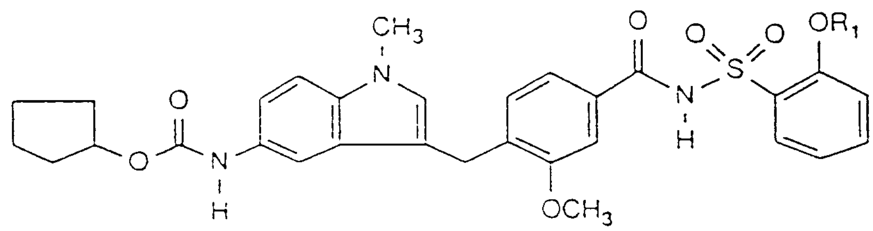

(IV),

où $R_1$ représente un groupe transformable en R, ou en un sel de celui-ci, on transforme $R_1$ en R et, si on le désire, on sépare chaque fois un mélange d'isomères, que l'on peut obtenir conformément au procédé, en ses composants et/ou on transforme un composé libre I que l'on peut obtenir conformément au procédé en un sel, ou on transforme un sel d'un composé I que l'on peut obtenir conformément au procédé, en composé I libre ou en un autre sel.

**12.** Procédé pour fabriquer une préparation pharmaceutique selon la revendication 9 ou 10, caractérisé en ce qu'on élabore la matière active, éventuellement en la mélangeant avec des adjuvants pharmaceutiques habituels, pour obtenir une préparation pharmaceutique.

**13.** Procédé selon la revendication 12 pour fabriquer une préparation pharmaceutique à activité antiallergique selon la revendication 10, caractérisé en ce que l'on choisit une matière active antiallergique.

**14.** Utilisation d'un composé selon une des revendications 1 à 6, sous forme libre ou sous forme de sel utilisable en pharmacie, pour fabriquer une préparation pharmaceutique.

**15.** Utilisation d'un composé selon une, des revendications 1 à 6, sous forme libre ou sous forme de sel utilisable en pharmacie, pour fabriquer une préparation pharmaceutiqued'une façon non chimique.

**16.** Utilisation d'un composé selon une des revendications 1 à 6 pour fabriquer un agent antiallergique.

**Revendications pour les Etats contractant suivants : ES, GR**

**1.** Procédé pour préparer un composé de formule

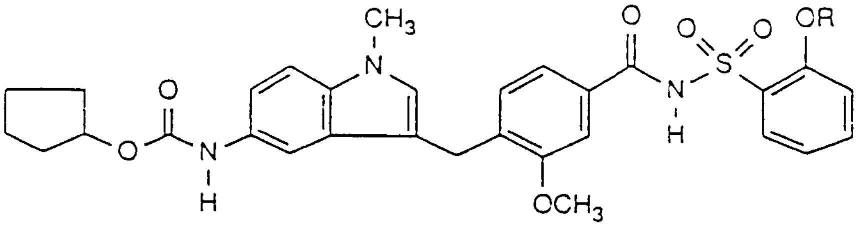

(I),

dans laquelle R représente un $C_2$-$C_4$-alk-1-ène-1-yle à chaîne droite, sous forme libre ou sous forme de sel, caractérisé en ce que

a) on fait réagir un composé de formule

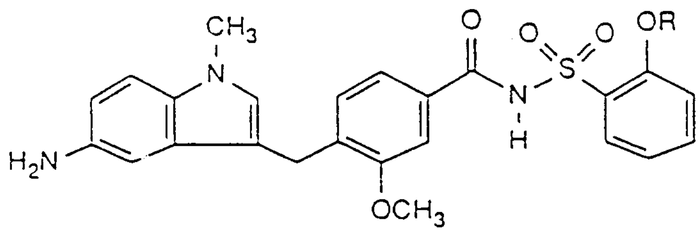

(II),

ou un sel de celui-ci avec un composé de formule

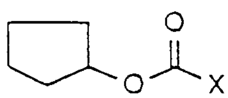

(III),

où X est un groupe éliminable, nucléofuge, avec séparation d'un composé H-X, ou

b) dans un composé de formule

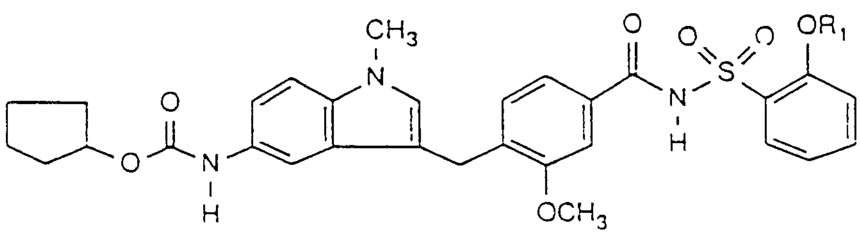

(IV),

où $R_1$ représente un groupe transformable en R, ou en un sel de celui-ci, on transforme $R_1$ en R et, si on le désire, on sépare chaque fois un mélange d'isomères, que l'on peut obtenir conformément au procédé, en ses composants et/ou on transforme un composé libre I que l'on peut obtenir conformément au procédé en un sel, ou on transforme un sel d'un composé I que l'on peut obtenir conformément au procédé, en composé I libre ou en un autre sel.

**2.** Procédé selon la revendication 1, caractérisé en ce que l'on prépare un composé de formule I, où R représente le vinyle, le (Z)-propéne-1-yle ou le (E)-propène-1-yle, sous forme libre ou sous forme de sel.

**3.** Procédé selon la revendication 1, caractérisé en ce que l'on prépare un composé de formule I, où R représente le vinyle, ou le (Z)-propène-1-yle, sous forme libre ou sous forme de sel.

**4.** Procédé selon la revendication 1, caractérisé en ce que l'on prépare le N-[4-(5-cyclopentyloxycarbonylamino-1-méthyl-indole-3-ylméthyl)-3-méthoxybenzoyl]amide de l'acide 2-vinyloxyberzène-sulfonique, sous forme libre ou sous forme de sel.

**5.** Procédé selon la revendication 1, caractérisé en ce que l'on prépare le N-[4-(5-cyclopentyloxycarbonylamino-1-méthyl-indole-3-ylméthyl)-3-méthoxybenzoyl]amide de l'acide (Z)-2-propène-1-yloxybenzènesulfonique, sous forme libre ou sous forme de sel.

**6.** Procédé selon la revendication 1, caractérisé en ce que l'on prépare le N-[4-(5-cyclopentyloxycarbonylamino-1-méthyl-indole-3-ylméthyl)-3-méthoxybenzoyl]amide de l'acide (E)-2-propène-1-yloxybenzènesulfonique, sous forme libre ou sous forme de sel.

**7.** Procédé pour fabriquer une préparation pharmaceutique, caractérisé en ce que

a) on fait réagir un composé de formule

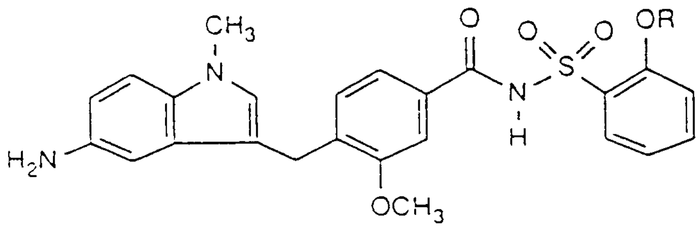

(II),

ou un sel de celui-ci avec un composé de formule

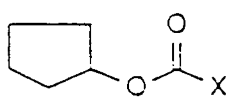

(III),

où X est un groupe éliminable, nucléofuge, avec séparation d'un composé H-X, ou

b) dans un composé de formule

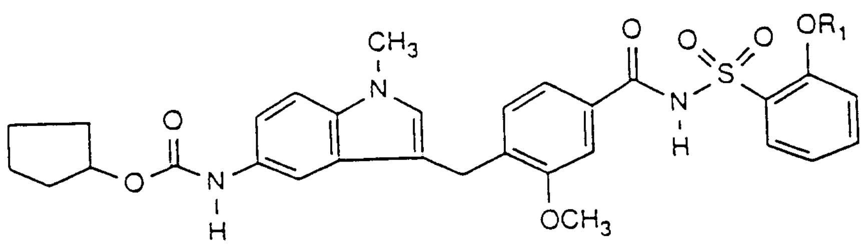

(IV),

où $R_1$ représente un groupe transformable en R, ou en un sel de celui-ci, on transforme $R_1$ en R et, si on le désire, on sépare chaque fois un mélange d'isomères, que l'on peut obtenir conformément au procédé, en ses composants et/ou on transforme un composé libre I que l'on peut obtenir conformément au procédé en un sel utilisable en pharmacie, ou on transforme un sel utilisable en pharmacie, que l'on peut obtenir conformément au procédé, d'un composé I en composé I libre ou en un autre sel utilisable en pharmacie, et l'on élabore un composé, obtenu de cette façon, de formule

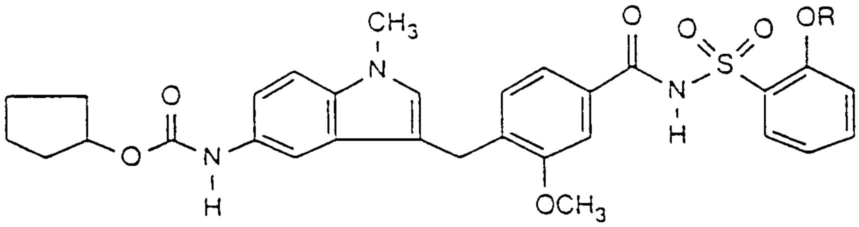

(I),

dans laquelle R représente un $C_2$-$C_4$-alk-1-ène-1-yle à chaîne droite, sous forme libre ou sous forme de sel utilisable en pharmacie, éventuellement en le mélangeant avec des adjuvants pharmaceutiques habituels, pour obtenir une préparation pharmaceutique.

**8.** Procédé pour fabriquer une préparation pharmaceutique, caractérisé en ce que l'on élabore un composé que l'on peut obtenir selon une des revendications 1 à 6, sous forme libre ou sous forme d'un sel utilisable en pharmacie, éventuellement en le mélangeant avec des adjuvants pharmaceutiques habituels, pour obtenir une préparation pharmaceutique.

**9.** Procédé selon la revendication 7 ou 8, pour fabriquer une préparation pharmaceutique ayant une activité antiallergique, caractérisé en ce que l'on choisit une matière active antiallergique.

**10.** Utilisation d'un composé que l'on peut obtenir selon une des revendications 1 à 6, sous forme libre ou sous forme d'un sel utilisable en pharmacie, pour fabriquer une préparation pharmaceutique.

**11.** Utilisation d'un composé que l'on peut obtenir selon une des revendications 1 à 6, sous forme libre ou sous forme d'un sel utilisable en pharmacie, pour fabriquer une préparation pharmaceutiqued'une façon non chimique.

**12.** Utilisation d'un composé que l'on peut obtenir selon une des revendications 1 à 6 pour fabriquer un agent antiallergique.